(19) Europäisches Patentamt
European Patent Office
Office européen des brevets

(11) EP 2 561 855 B1

(12) **EUROPEAN PATENT SPECIFICATION**

(45) Date of publication and mention of the grant of the patent:
**11.10.2017 Bulletin 2017/41**

(51) Int Cl.:
***A61K 8/26*** (2006.01) ***A61K 8/46*** (2006.01)
***A61Q 5/06*** (2006.01) ***A61Q 5/00*** (2006.01)
***A61K 8/22*** (2006.01)

(21) Application number: **11194638.0**

(22) Date of filing: **20.12.2011**

(54) **Method for chemically modifying the internal region of a hair shaft**

Verfahren zur chemischen Modifizierung des Innenabschnitts eines Haares

Procédé de modification chimique de la région interne de la tige d'un poil

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2011 EP 11151374**

(43) Date of publication of application:
**27.02.2013 Bulletin 2013/09**

(73) Proprietor: **The Procter & Gamble Company**
**Cincinnati, OH 45202 (US)**

(72) Inventor: **Flohr, Andreas**
**61476 Kronberg im Taunus (DE)**

(74) Representative: **Briatore, Andrea**
**Procter & Gamble Service GmbH**
**IP Department**
**Frankfurter Strasse 145**
**61476 Kronberg im Taunus (DE)**

(56) References cited:
**EP-A1- 2 295 029** **EP-A2- 0 173 195**
**WO-A1-2009/088520** **WO-A2-2007/127065**
**WO-A2-2010/014763** **US-A1- 2007 253 927**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 561 855 B1

## Description

FIELD OF THE INVENTION

In a first aspect, a method for chemically modifying the internal region of a hair shaft is provided. The method comprises applying an oxidising formulation to the hair; de-wetting the hair; applying a monomer composition to the hair, wherein the monomer composition comprises an ethylenic monomer having a molecular weight of 500 g/mole or less and a cosmetically acceptable carrier.

BACKGROUND OF THE INVENTION

Hair style retention traditionally has been accomplished by the use of styling products comprising polymers and other components that at least partially coat the hair and act on the surface of hair fibres. Several disadvantages are associated with this approach. Loss of hair style due to passage of time, elevated environmental humidity, excessive motion, etc. may cause a consumer to feel the need to refresh a hair style throughout the day. This often requires application of additional styling product(s). The benefits of traditional styling products also may be diminished when applied to hair to which conditioning agents also have been applied. In addition, application of materials to the surface of hair may compromise the natural feel and appearance of the hair and result in a dull appearance and/or a stiff or otherwise unpleasant feel. Furthermore, for certain hair types, it may not be possible to achieve desired hair styles with conventional styling products and treatments at all.

There is a need for consumers with damaged, thin, weak or limp hair to increase the volume and fullness of their hair, particularly consumers that utilise a blow drier. These consumers seek hair styles, treatments and products that allow them to perceive and project to others that they have thicker hair i.e. increased hair volume, bounciness and greater fullness. Improving hair movement and feel are also important desires of these consumers. Therefore, there is also a desire to enable consumers to be able to concurrently achieve increased volume and better hair feel, mobility, texture, healthy appearance and also increased control over their hairstyle. There is a need to allow these consumers to achieve such benefits without the need for back-combing, extensive blow drying, mousse products and/or hairsprays.

There is also a need for consumers with curly and/or unruly and/or frizzy hair to attain greater control of their hair, particularly consumers that utilise hair straighteners. These consumers seek hair styles, treatments and products that allow them to perceive and project to others that they have tamed hair and/or increased curl definition. In other words, all hairs are in place, the hair look is very defined, shiny and healthy-looking. These consumers may seek reduced volume and a straightening of the hair shafts leading to a more perfectly defined look. These consumers may usually resort to the utilisation of conventional means such as the application of styling gels and/or styling mousses. Hence, there is a need to allow these consumers to achieve such benefits without these consumers resorting to these conventional means.

Methods of, and compositions thereof for, chemically modifying the internal region of a hair shaft are known in the art. See for example US2008/0210253A1. Said methods may comprise the steps of applying to the hair a first composition comprising specific monomers and applying to the hair a second composition comprising an initiator. Said methods may result in increased rigidity of the hair shaft after multiple washings and/or wetting of the hair, reduced negative effects of moisture and/or humidity i.e. humidity resistance resulting in less frizziness or limpness, and said methods may require application of less styling products and/or may hold the style for longer periods of time.

Whilst said methods provide satisfactory results to the hair shaft, there is a constant need for providing methods resulting in further improved performance, efficacy and/or efficiency. As far as the improved performance is concerned, there is a specific need for providing improved desired hair shape retention and hairstyle durability. As far as efficiency is concerned, there is a need for improving the penetration of the monomers into the hair shaft and/or improving the polymerization of the monomers, for example increasing the lengths of the polymeric chains within the internal structure of the hair shaft. Furthermore, there is a need for improving the durability of the treatment such that the benefits last for a longer time. There is also a need for potentiating the efficacy of the method, for example such that less active(s) and/or composition(s)/formulation(s)/steps are required to achieve a similar performance. Moreover, there is a need for simplifying the application process, such that it can be carried out by either a trained hairdresser or a consumer at home on their own hair. In addition there is a need for better ensuring that the results are more predictable and reducing the variability of the end result between consumers with different hair types.

SUMMARY OF THE INVENTION

In a first aspect, the present invention relates to a method for chemically modifying the internal region of a hair shaft, wherein the method comprises:

(i) applying an oxidising formulation to the hair;
(ii) de-wetting the hair;
(iii) applying a monomer composition to the hair, wherein the monomer composition comprises an ethylenic monomer having a molecular weight of 500 g/mole or less and a cosmetically acceptable carrier, as defined in claim 1.

BRIEF DESCRIPTION OF THE DRAWINGS

Fig 1: Shows the disappearance of monomer over time as measured by proton nuclear magnetic resonance spectroscopy ($^1H$-NMR).
Fig 2: Shows the molecular weight of polymer that is formed when a monomer composition comprising $Al^{3+}$ cations is utilised and also when a monomer composition comprising $Sr^{2+}$ cations is utilised, as measured by gel permeation chromatography (GPC).
Fig 3A to 3E: Detection of the $^{14}C$-3-SPA in hair strands by micro-autoradiography.
Fig 4A to 4D: Detection of the $^{14}C$-3-SPA in hair strands by micro-autoradiography.

DETAILED DESCRIPTION OF THE INVENTION

In all embodiments of the present invention, all percentages are by weight of the total composition, unless specifically stated otherwise. All ratios are weight ratios, unless specifically stated otherwise. All ranges are inclusive and combinable. The number of significant digits conveys neither a limitation on the indicated amounts nor on the accuracy of the measurements.

All numerical amounts are understood to be modified by the word "about" unless otherwise specifically indicated. Unless otherwise indicated, all measurements are understood to be made at 25°C and at ambient conditions, where "ambient conditions" means conditions under about one atmosphere of pressure and at about 50% relative humidity. All such weights as they pertain to listed ingredients are based on the active level and do not include carriers or byproducts that may be included in commercially available materials, unless otherwise specified. Herein, "comprising" means that other steps and other ingredients which do not affect the end result can be added. This term encompasses the terms "consisting of" and "consisting essentially of". The compositions, methods, uses, kits, and processes of the present invention can comprise, consist of, and consist essentially of the elements and limitations of the invention described herein, as well as any of the additional or optional ingredients, components, steps, or limitations described herein.

The term "substantially free from" or "substantially free of" as used herein means less than about 1%, preferably less than about 0.8%, more preferably less than about 0.5%, still more preferably less than about 0.3%, most preferably about 0%, by total weight of the composition or formulation.

"Hair," as used herein, means mammalian hair including scalp hair, facial hair and body hair, more preferably hair on the human head and scalp. "Hair shaft" means an individual hair strand and may be used interchangeably with the term "hair."

"Internal region of the hair shaft," as used herein, means any non-surface portion of the hair shaft, including the inner portion of the cuticle and underneath the cuticle. "Non-surface portion" may be understood to mean that portion of the hair that is not in direct contact with the outside environment.

"Proximal to the scalp," as used herein, means that portion of an extended, or substantially straightened, hair shaft that is closer in distance to the scalp than to the end of the hair. Thus, about 50% of the hair would be considered proximal to the scalp, and about 50% of the hair would be distal to the scalp. "z cm proximal to the scalp" means a distance "z" along the hair, with one endpoint being on or directly adjacent to the scalp, and the second endpoint being measured "z" centimetres along the length of the extended or substantially straightened hair.

"Cosmetically acceptable," as used herein, means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives," as used herein, includes but is not limited to, amide, ether, ester, amino, carboxyl, acetyl, and/or alcohol derivatives of a given compound.

"Monomer," as used herein, means a discrete, non-polymerised chemical moiety capable of undergoing polymerisation in the presence of an initiator.

"Ethylenic monomer," as used herein, means a chemical species that contains an olefenic carbon-carbon double bond (C=C) and is capable of undergoing polymerization in the presence of an initiator.

"Polymer," as used herein, means a chemical formed from the polymerisation of two or more monomers. The term "polymer" as used herein shall include all materials made by the polymerisation of monomers as well as natural

polymers. Polymers made from only one type of monomer are called homopolymers. A polymer comprises at least two monomers. Polymers made from two or more different types of monomers are called copolymers. The distribution of the different monomers can be calculated statistically or block-wise - both possibilities are suitable for the present invention. Except if stated otherwise, the term "polymer" used herein includes any type of polymer including homopolymers and copolymers.

The term "hairstyling polymer" as used herein means hair-fixing polymers which form films on a surface. In the context of hair, this surface is the surface of individual hair fibres or a plurality thereof. The polymer causes them to be glued together to build welds, which are crosslinks that provide the hold benefit. In concert, these welds form a 'hairnet' to provide hair hold and volume benefits to the user. When the net of welds is effectively formed, the hold and volume benefits can last all day and offer good resistance to environmental humidity.

The term "molecular weight" or "M.Wt." as used herein refers to the number average molecular weight unless otherwise stated.

All percentages are calculated by weight unless otherwise stated.

"Chemically modify," or grammatical equivalents thereof, as used herein, means that a chemical moiety such as monomer and/or crosslinker and/or polymer, stably affixes to a second chemical moiety, for example, a keratin protein, another component of hair, and/or another monomer or crosslinker or polymer.

"Separately packaged," as used herein, means any form of packaging that prevents one composition or formulation from coming into physical contact, or admixing, with a second composition or formulation. "Separately packaged" may mean that the individual compositions/formulations are packaged in separate containers, or alternatively in a single container partitioned such that the compositions/formulations are not in physical contact.

"Relative humidity", as used herein, means the amount of water vapour carried in the air. "High relative humidity" herein refers to a relative humidity higher than that at ambient conditions i.e. at least about 70%. "Environmental humidity" as used herein relates to relative humidity that the consumer may be exposed to once the method of the present invention has been completed. An example of environmental humidity is that due to weather conditions. "Environmental humidity resistance", as used herein relates to the ability of the hair to better resist negative consequences of environmental humidity.

"Multivalent cation," as used herein, means an element having a net ionic charge of from 2+ to 7+.

"Stably affix" is understood to include both covalent and non-covalent forms of chemical bonds that once formed, remain unchanged through wetting, washing, styling and other types of hair treatment. In general, stably affixed chemical moieties may not be removed from the hair without damaging or substantially destroying the hair.

"Increased style retention and/or durability," as used herein, means that the hair style formed, shaped, and/or obtained after application of the composition of the present invention to the hair is maintained for a longer period of time relative to hair of the same being to which no composition has been applied.

"Increased appearance of volume," as used herein, means that the hair exhibits a visually noticeable greater volume, i.e., distance between the scalp and the outermost layer of hair style and/or distance between individual hairs, after application of a composition of the present invention relative to before a composition was applied.

"Increased resistance to moisture," as used herein, means that after application of a composition of the present invention, the hair fails to exhibit visually noticeable effects, such as loss of volume, loss of style, increase in frizz, etc. upon exposure to water vapour (i.e., relative humidity greater than about 50%) relative to before a composition has been applied.

"Kit," as used herein, means a packaging unit comprising a plurality of components. An example of a kit is, for example, a first composition and a separately packaged second composition. Another kit may comprise a first composition and an energy delivery device. A different kit may comprise three different types of separately packaged composition and a hair styling implement. A further kit may comprise application instructions comprising a method and a composition/formulation.

"Separately packaged," as used herein, means any form of packaging that prevents a first composition from coming into physical contact, or admixing, with a second composition. "Separately packaged" may mean that the individual compositions are packaged in separate containers, or alternatively in a single container partitioned such that the compositions are not in physical contact.

"Implement," as used herein, means a device used to facilitate application of a composition to the hair and/or manipulation of the hair. Examples of implements include, but are not limited to, a comb, a means for directed delivery (e.g., an applicator or tube), a covering for the hair (e.g., plastic bag, shower cap, etc.), and combinations thereof.

"Energy delivery device," as used herein, means any device used to deliver energy to keratinous tissue, including the hair and scalp. "Delivery of energy," means that the surface of the keratinous tissue is exposed to the energy emanating from the energy delivery device, where it may penetrate to the desired layers of the tissue, including the hair shaft and/or hair follicle. Energy includes but is not limited to energy in the form of light, heat, sound (including ultrasonic waves), electrical energy, magnetic energy, electromagnetic energy (including radiofrequency waves and microwaves), and combinations thereof.

In a first aspect, the present invention relates to method for chemically modifying the internal region of a hair shaft, wherein the method comprises:

(i) applying an oxidising formulation to the hair;
(ii) de-wetting the hair;
(iii) applying a monomer composition to the hair, wherein the monomer composition comprises an ethylenic monomer having a molecular weight of about 500 g/mole or less and a cosmetically acceptable carrier.

The inventors have answered the aforementioned needs by carefully selecting the specific combination of mutually compatible features such that the interaction therewith results in a method for chemically modifying the internal region of a hair shaft with improved performance. Particularly important benefits of the present invention include: reduced variability in the efficacy of the present invention between consumers with different hair types, and simplification of the process for applying the present invention.

Without being bound by theory, it is believed that a wide range of hair types exist in consumers, for example consumers may have highly damaged hair or undamaged hair. Further, it is believed that different hair types also contain different levels of redox active species that catalyze the decay of the initiator required to start the radical polymerization of an ethylenenically unsaturated monomer. Such redox active species could for example be residual amounts of metal (e.g. Fe, Co) present in the hair (e.g. from tap water), or cysteine, an amino acid found in keratin, a protein from which the hair shaft is made. The level at which those redox actives are present in human hair varies between different consumers depending upon how damaged their hair is. It is well established in polymer science that the molecular weight of a polymer made by radical polymerization is inversely proportional to the square root of the initial radical concentration. Therefore, it is believed that variability in the concentration of redox active species in consumer's hair leads to a variability of the molecular weight of the polymer created inside hair, which correlates with its wash fastness. A higher concentration of the redox active species will lead to lower molecular polymer created inside the hair, hence inferior wash fastness, and *vice versa.* Whilst the professional hair stylist may be able to judge the level of macroscopic damage of the hair which may have some degree of correlation to this amount of damage, it is neither efficient nor cost-effective to provide methods and compositions that are tailor-made to each consumer hair type. Consequently, as embodied in the present invention, the inventors have surprisingly found that the application of an oxidising formulation to the hair prior to the application of the monomer composition results in improved performance. It is believed that this oxidising step results in the normalisation of concentration of the redox active species between hair types. A reduction step may also reduce the variability of the different hair types as well, but would at the same time also demand a significant reduction in initiator concentration to keep the initial radical concentration low. However, the initiator concentration would need to be lowered to a level at which it cannot be handled under realistic application conditions.

The inventors have also surprisingly found that it is not a requirement to have a reducing step prior to application of the monomer composition, as is often conventionally taught, in order to reduce the disulfide bridges and open up the cuticle. Excellent benefits result when hair is treated according to the present invention without using a reduction step prior to the application of the monomer composition.

It has also been found that the monomer composition can be provided to the consumer and/or stylist in ready-to-use form. In other words, without the need for the monomer composition to be mixed with at least one other formulation/composition, for example a composition comprising an initiator, salt, oxidising agent, acid, base, or catalyst, prior to application onto hair. This represents a significant improvement in the method for applying the present invention onto hair, in terms of reduced application time, transport effort, carbon footprint and cost effectiveness.

The present invention pertains to compositions and methods for chemically modifying the internal region of the hair shaft, more preferably for forming polymer in the internal region of the hair shaft. Without being bound by theory, it is believed that polymer on the external surface of the hair shaft would be more easily washed off whereas polymer inside the hair shaft, for example, underneath the cuticle, would be better protected and hence be substantially more durable. Fig 1 and 2 as detailed herein demonstrate that polymer is formed by the actives utilised in the present invention.

The features of the method according to the first aspect, as well as the other aspects and other relevant components, are described in detail hereinafter.

The method of the present invention comprises (i), (ii), and (iii), as described herein, which indicate a specific order i.e. (i) followed by (ii) followed by (iii).

The method of the present invention comprises applying an oxidising formulation to the hair. The oxidising formulation comprises an oxidising agent. The oxidising agent may be selected from the group consisting of: peroxides, preferably hydrogen peroxide; persulfates, preferably potassium persulfate or sodium persulfate; and mixtures thereof. Another composition or formulation as mentioned herein, or a plurality thereof, may comprise at least one oxidising agent. In an embodiment, the monomer composition does not comprise an oxidising agent. In an embodiment, the oxidising formulation is substantially free of hair colouring agents, or substantially free of oxidative dyes and/or direct dyes.

The oxidising agent may be present in an amount of from about 0.01% to about 15%, by total weight of the

composition/formulation. When a persulfate oxidising agent is used, it may be in powder form and mixed as a liquid immediately prior to application onto hair. The final amount of persulfate in the composition/formulation may be from about 0.5% to about 2%, more preferably 0.8% to about 1.2%, by total weight of the composition/formulation. When the oxidising agent is a peroxide, the peroxide may be present in an amount of from about 0.5% to about 5%, preferably from about 1% to about 4%, more preferably from about 1.3% to about 3%, most preferably from about 1.5% to about 3%, by total weight of the composition or formulation.

In an embodiment, after applying the oxidising formulation to the hair but prior to de-wetting the hair, the oxidising formulation is allowed to remain on the hair for a period of time y, wherein time y is from about 1 min to about 120 mins, preferably from about 2 mins to about 45 mins, more preferably from about 3 mins to about 20 mins, most preferably from about 4 mins to about 10 mins.

When the oxidising formulation comprises peroxide, the oxidising formulation may comprise a buffer system to stabilise the pH. Suitable buffers may also act as chelating agents. Chelation of transition metals, for example copper or iron from pipes which might be present in trace amounts in tap water, is important because peroxides are sensitive to cleavage by transition metals. In the absence of a buffer system the transition metal may cleave the peroxide, deactivating it.

Typical buffer systems comprise a strong acid and its weak conjugate base or a weak base and its conjugate acid. An example of a suitable buffer system is phosphoric acid and disodium phosphate. Another example of a suitable buffer system is citric acid and sodium hydroxide. In an embodiment, the monomer composition comprises a buffer system.

The method of the present invention comprises de-wetting the hair. The de-wetting the hair comprises the application of an absorbent material to the hair such that wetness is transferred from the hair to the absorbent material and wherein the wetness comprises the cosmetically acceptable carrier. The absorbent material may be selected from the group consisting of: towel, absorbent paper, and combinations thereof. In an embodiment, the de-wetting the hair comprises allowing moisture to evaporate from the hair wherein the moisture comprises the cosmetically acceptable carrier. In an embodiment, the de-wetting the hair comprises towel drying the hair such that the oxidising formulation no longer drips from the hair. In an embodiment, the de-wetting the hair comprises removing superficial oxidising formulation from the hair. In an embodiment, the de-wetting the hair does not comprise rinsing the oxidising formulation from the hair. The de-wetting the hair may last for time z, wherein time z is from about 1 min to about 120 mins, preferably from about 2 mins to about 45 mins, more preferably from about 3 mins to about 20 mins, most preferably from about 4 mins to about 10 mins.

The method of the present invention comprises applying a monomer composition to the hair. The monomer composition comprises an ethylenic monomer having a molecular weight of about 500 g/mole or less. In an embodiment, the ethylenic monomer is selected from the group consisting of: mesaconic acid, 2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2-methylenebutyrate, methacryloyl-L-lysine, N-(2-hydroxypropyl)methacrylamide, 2-acrylamidodiglycolic acid, 2-ethoxyethyl acrylate, 2-butoxyethyl acrylate, N-isopropylmethacryalmide, 2-aminoethyl methacrylate, 2-bromoethyl acrylate, 3-(dimethylamino)propyl acrylate, (3-acrylamidopropyl)trimethyl ammonium salt, [2-(acryloyloxy)ethyl]-trimethylammonium salt, alkylacetamidoacrylate, sulfoalkyl(meth)acrylate, 3-sulfopropyl acrylate, 3-sulfopropyl methacrylate, and salts, isomers, derivatives and mixtures thereof. In an embodiment, the monomer composition comprises at least one ethylenic monomer, selected from the group cited above, as the sole ethylenic monomer(s). In an embodiment, the ethylenic monomer is selected from the group consisting of 3-sulfopropyl acrylate, 3-sulfopropyl methacrylate, and salts, derivatives and mixtures thereof. In an embodiment, the only ethylenic monomer(s) present are selected from the group consisting of 3-sulfopropyl acrylate, 3-sulfopropyl methacrylate, and salts, derivatives and mixtures thereof. In an embodiment, the sole ethylenic monomer is 3-sulfopropyl acrylate. In an embodiment, the ethylenic monomer is 3-sulfopropyl acrylate, which is added to the composition as 3-sulfopropyl acrylate potassium salt.

In another embodiment, the ethylenic monomer is selected from the group consisting of: acrylic acid, sodium acrylate, potassium acrylate, calcium acrylate, monoethanolamine acrylate, 3-hydroxypropyl acrylate, 2,5-butylaminoethyl acrylate, methacrylic acid, sodium methacrylate, potassium methacrylate, calcium methacrylate, monoethanolamine methacrylate, 2-N,N-dimethylaminoethyl acrylate, glycidyl methacrylate, 2-dimethylamino ethyl methacrylate, 2-hydroxypropyl methacrylate, 3-hydroxypropyl methacrylate, 2,4-dihydroxybutyl methacrylate, 2,3-epoxybutyl methacrylate, 2-t-butylaminoethyl methacrylate, 2-(2-diethylamino)ethyl methacrylate, ethylene glycol mono methacrylate, itaconic acid (and salts thereof), vinyl pyridine, resorcinol, and mixtures thereof.

The molecular weight of the ethylenic monomer is important because of the need for the monomer to penetrate into the hair shaft prior to polymerisation. Large and/or bulky monomers would penetrate less easily into the hair shaft. In an embodiment, the ethylenic monomer has a molecular weight of from about 50 g/mole to about 500 g/mole, preferably from about 75 g/mole to about 400 g/mole, more preferably from about 100 g/mole to about 400 g/mole, even more preferably from about 150 g/mole to about 300 g/mole. In an embodiment, the ethylenic monomer does not have a

molecular weight of below about 50 g/mole, preferably below about 75 g/mole, more preferably below about 100 g/mole, even more preferably below about 150 g/mole, nor above about 500 g/mole, preferably nor above about 400 g/mole, even more preferably nor above about 300 g/mole.

The ethylenic monomer may be present in an amount of from about 0.1% to about 20%, preferably from about 1% to about 15%, more preferably from about 5% to about 14%, even more preferably from about 7% to about 13%, most preferably from about 11% to about 12.5%, by total weight of the monomer composition.

In an embodiment, two or more different ethylenic monomers are present in the monomer composition. The resultant polymers may be copolymers.

In an embodiment, the monomer composition is substantially free of oxidising agents and/or initiators. In another embodiment, the monomer composition is substantially free of oxidising agents selected from the group consisting of: peroxides, preferably hydrogen peroxide; persulfates, preferably potassium persulfate or sodium persulfate; and mixtures thereof. In another embodiment, the monomer composition is substantially free of an alpha-methylene lactone compound.

In another embodiment, the monomer composition is in ready-to-use form, wherein ready-to-use form means that no pre-mixing is required prior to application onto hair. In another embodiment, the monomer composition is substantially free of at least one of the following: a reducing agent, a transition metal.

According to the first aspect, the method of the present invention may further comprise one or more of the following: allowing the monomer composition to remain on the hair for a period of time x, wherein the time x is from about 1 min to about 120 mins; rinsing the hair; washing the hair. The time x may be from about 5 mins to about 100 mins, more preferably from about 10 mins to about 90 mins, most preferably from about 20 to about 60 mins.

A composition or formulation as described herein, or a plurality thereof, comprises a cosmetically acceptable carrier. The composition or formulation, for example the monomer composition or oxidising formulation, may comprise from about 60% to about 99.9%, alternatively from about 70% to about 95%, and alternatively from about 80% to about 90%, of a cosmetically acceptable carrier, by total weight of the composition or formulation. Cosmetically acceptable carriers suitable for use include, for example, those used in the formulation of tonics and gels. Cosmetically acceptable carrier may comprise water; silicones such as volatile silicones, amino or non-amino silicone gums; organic compounds such as $C_2$-$C_{10}$ alkanes, acetone, methyl ethyl ketone, volatile organic $C_1$-$C_{12}$ alcohols, esters of $C_1$-$C_{20}$ acids and of $C_1$-$C_8$ alcohols such as methyl acetate, butyl acetate, ethyl acetate, and isopropyl myristate, dimethoxyethane, diethoxyethane, $C_{10}$-$C_{30}$ fatty alcohols such as lauryl alcohol, cetyl alcohol, stearyl alcohol, and behenyl alcohol; $C_{10}$-$C_{30}$ fatty acids such as lauric acid and stearic acid; $C_{10}$-$C_{30}$ fatty amides such as lauric diethanolamide; $C_{10}$-$C_{30}$ fatty alkyl esters such as $C_{10}$-$C_{30}$ fatty alkyl benzoates; hydroxypropylcellulose, and mixtures thereof. In an embodiment, the carrier comprises water, fatty alcohols, volatile organic alcohols, and mixtures thereof. In a most preferred embodiment, the carrier is water.

The monomer composition may comprise a crosslinker having a molecular weight suitable to penetrate the hair shaft. The purpose of the crosslinker is to covalently bond the monomer to the hair, monomer to other monomer, and polymer to other polymer.

The molecular weight of the crosslinker may be about 500 g/mole or less, preferably from about 100 g/mole to about 500 g/mole, more preferably from about 100 g/mole to about 400 g/mole, even more preferably from about 150 g/mol to about 400 g/mole.

The crosslinkers may be selected from the group consisting of: 1,4-bisacryloylpiperazine, methylenebisacrylamide, ethylenebisacrylamide, divinylbenzene, poly-ethyleneglycol di(meth)acrylate, ethylene glycol di(meth)acrylate, 1,3-butanediol di(meth)acrylate, 1,4-butanediol di(meth)acrylate, neopentyl glycol di(meth)acrylate, bis[2-(methacryloyloxy)ethyl] phosphate, N,N'-bis(acryloyl)cystamine, N,N-diallylacryalmide, triallyl cyanurate, 3-(acryloyloxy)-2-hydroxypropyl methacrylate, and mixtures thereof.

In an embodiment, the ratio of the weight percentage of the ethylenic monomer to the weight percentage of the crosslinker (i.e. ethylenic monomer:crosslinker) is from about 50:1 to about 10:1, alternatively from about 40:1 to about 10:1, and alternatively from about 20:1 to about 10:1.

The present invention relates to a method for chemically modifying the internal region of the hair shaft. In an embodiment, the chemically modifying is selected from the group consisting of: the formation of a polymer in the internal region of the hair shaft; the modification of the internal region of the hair shaft with a polymer; and combinations thereof. In an embodiment, the polymerisation that occurs is free radical polymerisation.

In an embodiment, at least one performance benefit as described herein, which the present invention provides, is still noticeable to at least about 40% of consumers after about 15 washes, preferably to at least about 45% of consumers, more preferably to at least about 50% of consumers, even more preferably to at least about 55% of consumers, most preferably to at least about 60% of consumers, optimally to at least about 65% of consumers, more optimally to at least about 70% of consumers The consumers are selected from the group consisting of: consumers that regularly style their hair and have thin and/or limp hair, and consumers that regularly style their hair and have thick and/or unruly hair.

A composition or formulation as mentioned herein, or a plurality thereof, may further comprise a monomer that does not penetrate into the internal region of the hair shaft, which will be referred to as a non-penetrating monomer

hereinafter. Reasons for non-penetration include hydrophobicity, insolubility, too large a molecular weight i.e. greater than about 500 g/mol, and also highly reactive monomers that may polymerise before reaching the hair shaft. It is hence implicit that such non-penetrating monomers are not capable of chemically modifying the internal region of a hair shaft. However, non-penetrating monomers may be capable of chemically modifying the external surface of the hair shaft and that this modification may be capable of enduring a plurality of washings/shampooings. This external modification may enhance the benefits of the present invention. Non-penetrating monomers include those of cyanoacrylate chemistry and reactive silicones. Such non-penetrating monomers include those disclosed in US7357921B2, US 7780742, US 7740664.

A composition or formulation as described herein, or a plurality thereof, may further comprise a viscosity-increasing means selected from the group consisting of viscosity-increasing agents and viscosity-increasing systems. Viscosity is important when the composition is in the form of gel, cream, lotion, emulsion etc because it prevents the composition from sliding off the hair. However, lower viscosities allow actives to penetrate/diffuse more easily into the internal region of the hair shaft. The viscosity of the composition when it is in the form of a gel is: preferably from about 500 mPa·s to about 7000 mPa·s, more preferably from about 1000 mPa·s to about 5000 mPa·s, even more preferably from about 1500 mPa·s to about 4500 mPa·s, most preferably from about 1900 mPa·s to about 4000 mPa·s, measured with a Brookfield Viscosimeter RVDV III Ultra CP 52 at 25 °C and 1 rpm.

The viscosity-increasing agent may be selected from the group consisting of non-ionic thickeners, cationic thickeners, anionic thickeners, amphoteric thickeners, and mixtures thereof; preferably non-ionic thickeners, anionic thickeners, and mixtures thereof. The viscosity-increasing agent may be present in the composition in an amount of from about 0.1% to about 10%, preferably about 0.2% to about 5.0%, by total weight of the composition.

Non-ionic or anionic thickeners (or mixtures thereof) are preferred for the monomer composition due to the typically anionic chemistry of polymerised ethylenic monomer. Non-ionic or anionic thickeners are less likely to interact directly with the formed polymer and hence the formation of insoluble complexes or precipitates is also less likely. The viscosity-increasing means is also preferably stable at the required pH and does not substantially affect the active levels of ethylenic monomer. The viscosity-increasing agent may be a crosslinked or a non-crosslinked polymer.

In an embodiment, the viscosity-increasing agent is a hydrophobically-modified polyacrylate polymer. Such hydrophobically-modified polyacrylate polymers are particularly suitable when the composition/formulation is created by the addition of at least one salt. The monomer composition may comprise from about 0.5% to about 1.5% of the hydrophobically-modified polyacrylate polymer, by total weight of the monomer composition. Suitable hydrophobically-modified polyacrylate polymers include: acrylates/C10-C30 alkylacrylates copolymers such as Ultrez® 20/21 from Lubrizol, and Permulen® TR1 from Lubrizol; acrylates/beheneth-25 methacrylate copolymers such as Aculyn® 28 from Rohm & Haas; acrylates/ceteth-20 itaconate copolymers such as Structure® 3001 or 2001 from Akzo Nobel.

In an embodiment, the viscosity-increasing agent is a non-crosslinked associative thickening polymer. The monomer composition may comprise from about 0.5% to about 3% of the non-crosslinked associative thickening polymer, by total weight of the monomer composition. Suitable associative thickeners include polyurethane-based polymers such as polyurethane-30 e.g. LuvigelSTAR® from BASF. Also EO-PO-block copolymers may be useful, for example Pluronics® from BASF.

In an embodiment, the viscosity-increasing agent comprises at least one polysaccharide, preferably at least one heteropolysaccharide. The total polysaccharide present in the monomer composition may be from about 0.2% to about 5%, more preferably from about 0.5% to about 4%, by total weight of the monomer composition. Suitable polysaccharides and heteropolysaccharides include starches and derivatives thereof, e.g. mono- or di-esters with phosphoric acid, cellulose types and their derivatives, xanthan gums, carrageenans. Preferred heteropolysaccharides include xanthan gum such as Keltrol® T from Kelco, and Natrosol® 250 HHR from Herkules. In an embodiment, the polysaccharide is selected from the group consisting of hydroxyethylcellulose, hydroxypropylcellulose, xanthan gum, carrageenans, and mixtures thereof. Xanthan gums and derivatives thereof are present in an amount of from about 0.2% to about 1.5%, more preferably about 0.5% to about 0.9%, by total weight of the monomer composition. Starches and derivatives thereof are present in an amount of from about 3% to about 4% by total weight of the monomer composition. A preferred starch is hydroxypropyl starch phosphate such as Structure® XL from National Starch. In an embodiment, the monomer composition comprises two different heteropolysaccharide viscosity-increasing agents.

In an embodiment, a viscosity-increasing system is employed. The viscosity-increasing system may employ a viscosity-increasing agent as defined above. Alternatively or additionally, the viscosity-increasing system comprising a non-polymer system which provides internal structure to the product. The non-polymer system is selected from the group consisting of: a surfactant system preferably comprising lauryl ether sulphate plus sodium chloride; a liquid crystal system for example a cationic conditioner made of fatty alcohol plus cationic surfactant; an emulsion of the water in oil (w/o) type; an emulsion of the oil in water (o/w) type; an inorganic thickening system, for example bentonites, montmorillonites. In another embodiment, the viscosity-increasing system is free of a polymeric viscosity-increasing agent or polymeric thickener.

In an embodiment, a composition or formulation as mentioned herein, or a plurality thereof, may comprise a cation, wherein the cation is selected from the group consisting of inorganic cations having a charge density of about

0.05 charge/picometre or more, preferably about 0.052 charge/picometre or more. In an embodiment the inorganic cation is a metal. In an embodiment, the cation may not be selected from inorganic cations having a charge density of about 0.04 charge/picometre or less, preferably less than about 0.05 charge/picometre. In an embodiment, the cation is selected from the group consisting of inorganic cations having a charge density of about 0.050 charge/picometre to about 0.090 charge/picometre, preferably from about 0.052 charge/picometre to about 0.080 charge/picometre, more preferably to about 0.070 charge/picometre, even more preferably to about 0.060 charge/picometre, most preferably to about 0.053 charge/picometre.

The presence, in the monomer composition and/or the oxidising formulation of the present invention, of an inorganic cation having a charge density of about 0.05 charge/picometre or more results in superior performance. Without being bound by theory, it is believed that the inorganic cation, due to its positive charge, can bind to a plurality of anionic ethylenic monomers, either when the ethylenic monomer is unpolymerised or after polymerisation when it is a unit of the polymer. For example, the inorganic cation may bind to two or more different polymer chains that carry an anionic charge simultaneously. The benefits of such binding effects include: increased sequestering of ethylenic monomer and polymer into greater proximity to each other resulting in longer polymer chains and/or ionic cross-linking of neighboured polymer chain segments after polymerization. Inorganic cations having a higher charge density are able to bind and out-compete any other cation with a lower charge density for the negatively charged binding site.

The charge density of an ion is calculated by dividing the charge by the ionic radius which results in charge per picometre, the charge density. For example, $Sr^{2+}$ has an ionic radius of 127 pm and a charge of 2. Consequently, the charge per picometre is 0.0157 charge/pm. Tables of ionic radii tables can be found in common inorganic chemistry textbooks, for example Atkins P.W., Physical Chemistry, 6th Edition, 2001. Ion radii of common metals can be found in Table I.

Table I

| Metal | Charge | Picometre (pm) per charge | Charge density (charge/pm) | Ion radius (pm) |
|---|---|---|---|---|
| K (I) | 1+ | 133.00 | 0.0075 | 133 |
| Cd (I) | 1+ | 114.00 | 0.0088 | 114 |
| Na (I) | 1+ | 98.00 | 0.0102 | 98 |
| Cu(I) | 1+ | 96.00 | 0.0104 | 96 |
| Li (I) | 1+ | 78.00 | 0.0128 | 78 |
| Ba (II) | 2+ | 71.50 | 0.0140 | 143 |
| Sr (II) | 2+ | 63.50 | 0.0157 | 127 |
| Ca (II) | 2+ | 53.00 | 0.0189 | 106 |
| Cd (II) | 2+ | 51.50 | 0.0194 | 103 |
| Sn (II) | 2+ | 46.50 | 0.0215 | 93 |
| Mn (II) | 2+ | 45.50 | 0.0220 | 91 |
| Ge (II) | 2+ | 45.00 | 0.0222 | 90 |
| Ag (II) | 2+ | 44.50 | 0.0225 | 89 |
| Cr (II) | 2+ | 42.00 | 0.0238 | 84 |
| Zn (II) | 2+ | 41.50 | 0.0241 | 83 |
| Fe (II) | 2+ | 41.00 | 0.0244 | 82 |
| Co (II) | 2+ | 41.00 | 0.0244 | 82 |
| La (III) | 3+ | 40.67 | 0.0246 | 122 |
| Ni (II) | 2+ | 39.00 | 0.0256 | 78 |
| Mg (II) | 2+ | 39.00 | 0.0256 | 78 |
| Cu (II) | 2+ | 36.00 | 0.0278 | 72 |
| Ce (III) | 3+ | 35.67 | 0.0280 | 107 |
| Au (III) | 3+ | 30.33 | 0.0330 | 91 |

(continued)

| Metal | Charge | Picometre (pm) per charge | Charge density (charge/pm) | Ion radius (pm) |
|---|---|---|---|---|
| Ce (IV) | 4+ | 23.50 | 0.0426 | 94 |
| Mn (III) | 3+ | 23.33 | 0.0429 | 70 |
| Fe (III) | 3+ | 22.33 | 0.0448 | 67 |
| Zr (IV) | 4+ | 21.75 | 0.0460 | 87 |
| V (III) | 3+ | 21.67 | 0.0462 | 65 |
| Cr (III) | 3+ | 21.33 | 0.0469 | 64 |
| Cr (III) | 3+ | 21.33 | 0.0469 | 64 |
| Co (III) | 3+ | 21.33 | 0.0469 | 64 |
| NI (III) | 3+ | 20.67 | 0.0484 | 62 |
| Al (III) | 3+ | 19.00 | 0.0526 | 57 |
| Sn (IV) | 4+ | 18.50 | 0.0541 | 74 |
| Se (IV) | 4+ | 17.25 | 0.0580 | 69 |
| V (IV) | 4+ | 15.25 | 0.0656 | 61 |
| Cr (IV) | 4+ | 14.00 | 0.0714 | 56 |
| Ge (IV) | 4+ | 13.25 | 0.0755 | 53 |
| Mn (IV) | 4+ | 13.00 | 0.0769 | 52 |
| V (V) | 5+ | 11.80 | 0.0847 | 59 |

Without being bound by theory, it is believed that unfavourable redox reactions with the chemistry of other hair treatments (such as permanent oxidative hair colouring, semi-permanent hair colouring, hair dyeing, hair bleaching, or permanent waving of the hair) are less likely to occur when the cation is less able to lose or gain electrons. Unfavourable redox reactions are less likely to occur if the inorganic cation is selected from metals that can only exist in two oxidation states other than oxidation state "0", more preferably in only one oxidation state other than oxidation state "0". Copper, for example, can only exist in a total of three different oxidation states, namely $Cu^0$, $Cu^{1+}$ and $Cu^{2+}$. Manganese, for example, can only exist in a total of four different oxidation states, namely $Mn^0$, $Mn^{2+}$, $Mn^{3+}$, $Mn^{4+}$. Aluminium, for example, can only exist in a total of two oxidation states, namely $Al^0$ and $Al^{3+}$. In an embodiment, the inorganic cation has a charge of at least 2+, more preferably at least 3+. In an embodiment, the inorganic cation is not a transition metal. In an embodiment, the inorganic cation is not a metal capable of cleaving hydrogen peroxide. In a preferred embodiment, the inorganic cation is $Al^{3+}$.

The molar ratio of the cation to the monomer (i.e. cation:monomer) may be from about 1:10 to about 2:1, preferably from about 1:5 to about 3:2, more preferably from about 1:2 to about 1:1, even more preferably from about 1:3 to about 1:1. The molar amount of cation is preferred to be in excess of the molar amount of counterion of the ethylenic monomer when the ethylenic monomer is added into the monomer composition in the form of a salt.

The cation may be present in the monomer composition, the oxidising formulation, the finishing formulation, and/or a combination thereof. In an embodiment, the monomer composition and/or the oxidising formulation further comprises a cation, wherein the cation is not selected from inorganic cations having a charge density of about 0.04 charge/picometre or less, preferably less than about 0.05 charge/picometre. In an embodment, the monomer composition and/or the oxidising formulation further comprises a cation and an anion; wherein the cation is selected from the group consisting of inorganic cations having a charge density of 0.05 charge/picometre or more. In an embodiment, the oxidising formulation comprises a cation, wherein the cation is selected from the group consisting of inorganic cations having a charge density of about 0.05 charge/picometre or more, and the cation is present in an amount of from about 0.001% to about 2%, preferably about 0.01% to about 1%, more preferably about 0.04% to about 0.2%, by total weight of the oxidising formulation.

In an embodiment, a composition or formulation, or a plurality thereof, may comprise an anion. The anion may be selected from the group consisting of sulfate, sulfonate, phosphate, nitrate, chloride, citrate, lactate, formate, and mixtures thereof. Preferred anions are selected from the group consisting of sulfate, sulfonate, and mixtures thereof. In a most preferred embodiment, the anion is sulfate.

In an embodiment, the molar ratio of the cation to the anion (cation:anion) is from about 1:5 to about 5:1, preferably from about 1:4 to about 3:1, most preferably from about 2:3 to about 3:2. Where the inorganic metal cation is $Al^{3+}$ and the anion is sulfate, the molar ratio of the cation to the anion is about 2:3. The anion may be present in the monomer composition, the oxidising formulation, the finishing formulation, or a combination thereof.

In an embodiment, a composition or formulation as described herein, or a plurality thereof, may be created via the addition of a salt. In an embodiment, a composition or formulation as described herein, or a plurality thereof, comprises a salt. The salt may comprise an anion and a cation having a charge density of about 0.05 charge/picometre or more. In an embodiment, the salt may comprise the ethylenic monomer.

The pH of the compositions and formulations of present invention, when applied to hair directly, must be suitable for application onto human hair i.e. the compositions and formulations must be cosmetically acceptable. Furthermore, the pH of the present invention must be suitable to ensure that all components of the composition or formulation are dissolved and stable. The monomer composition may have a pH of from about 2.0 to about 9.0. In an embodiment, the pH is from about 2.5 to about 7.5, preferably from about 4.0 to about 7.0, more preferably from about 4.0 to about 6.9. In an embodiment, the pH is from about 4.5 to about 6.7, preferably from about 5.0 to about 6.6, more preferably from about 5.2 to about 6.5, even more preferably from about 5.3 to about 6.5, most preferably from about 5.5 to about 6.5, optimally from about 5.5 to about 6.0. In an embodiment, the composition does not have a pH of about 7.0 or more.

A composition or formulation as described herein, or a plurality thereof, may comprise an initiator. Preferred initiators include: peroxidisulfates, peroxides, peracids, perborates, peroxyesters, sodium peroxydisulfate, benzoyl peroxide, peracetic acid, azo initiators, and mixtures thereof. Transition metal catalysts or metal initiators that can exist in a total of three oxidation states, other than oxidation state "0", are not suitable for the present invention. Preferably the monomer composition is substantially free from transition metal catalyst or metal initiator that can exist in a total of three oxidation states, other than oxidation state "0", more preferably the monomer composition is substantially free from a transition metal catalyst or metal initiator that can exist in a total of two oxidation states, other than oxidation state "0". In an embodiment, the initiator is provided to the hair via an energy delivery device by applying said device proximal to the scalp. A suitable initiator may include light energy.

Following treatment with the monomer composition of the present invention, a finishing composition may be applied to the hair. The finishing composition may comprise a hair conditioning agent. The hair conditioning agent is typically a cationic polymer that provides softness to the hair or that repair damaged hair. Conditioning polymers generally provide a film on the hair that is smooth and not tacky. Typically, the conditioning polymers are cationic but may also be nonionic, zwitterionic, and amphoteric. The hair conditioning agent may also be an oily and/or a silicone compound. Patent application WO 2009/107062 A2 discloses conditioning agents and cationic polymers, which may be suitable for the finishing formulation as described herein. Also potentially suitable for use in the compositions herein are the conditioning agents described by the Procter & Gamble Company in U.S. Pat. Nos. 5,674,478, and 5,750,122. Also potentially suitable for use herein are those conditioning agents described in U.S. Pat. Nos. 4,529,586 (Clairol), 4,507,280 (Clairol), 4,663,158 (Clairol), 4,197,865 (L'Oreal), 4,217, 914 (L'Oreal), 4,381,919 (L'Oreal), and 4,422, 853 (L'Oreal).

In an embodiment, the finishing formulation comprises an oxidising agent. The oxidising agent may be present in an amount of from about 0.01% to about 15%, by total weight of the finishing formulation.

A composition or formulation as described herein, or a plurality thereof, may comprise a reducing agent, which may be useful for allowing reducing disulfide bonds and improved penetration into the hair shaft. Examples of suitable reducing agents include, but are not limited to, sodium thioglycolate, anhydrous sodium thiosulfate, powdered sodium metabisulfite, thiourea, ammonium sulfite, thioglycolic acid, thiolactic acid, ammonium thiolactate, glyceryl monothioglycolate, ammonium thioglycolate, thioglycerol, 2,5-dihydroxybenzoic acid, diammonium dithioglycolate, strontium thioglycolate, calcium thioglycolate, zinc formosulfoxylate, isooctyl thioglycolate, *D*/*L*-cysteine, monoethanolamine thioglycolate, phosphines, and mixtures thereof.

A reducing formulation comprises the reducing agent, and wherein the reducing formulation may be separately packaged from other compositions or formulations. The reducing formulation may comprise from about 1% to about 12%, alternatively from about 4% to about 10%, and alternatively from about 8% to about 10%, of a reducing agent, by total weight of the reducing formulation.

A composition or formulation as described herein, or a plurality thereof, may further comprise one or more optional components known or otherwise effective for use in hair care or personal care products, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004, both of which are incorporated by reference herein in their entirety. Some non-limiting examples of such optional components are disclosed below, and include plasticizers, surfactants (which may be anionic, cationic, amphoteric or nonionic), neutralizing agents, propellants, hair conditioning agents (e.g., silicone fluids, fatty esters, fatty alcohols, long chain hydrocarbons, cationic surfactants, etc.), emollients, lubricants and penetrants such as various lanolin compounds, vitamins, proteins, preservatives, dyes, tints, bleaches, reducing agents and other colorants, sunscreens, gelling agents, physiologically active compounds for treating the hair or skin (e.g., anti-dandruff actives, hair growth actives), non-polymeric thickeners including clays, and perfume.

Oily compounds may aid the penetration of the ethylenic monomer into the skin and/or scalp, which is not preferred. In an embodiment, the monomer composition is substantially free from oily compounds. In another embodiment, the monomer composition and the oxidising formulation are substantially free from oily compounds.

The monomer composition of present invention may be in different product forms. It may be in a form selected from the group consisting of a gel, an emulsion, a cream, a spray, a lotion, or a mousse. Preferred forms are a gel, a cream or a lotion, more preferably a gel, most preferably the monomer composition is in the form of a gel that has the appearance of an emulsion. The oxidising formulation may be in the form of gel, an emulsion, a cream, spray, lotion, mousse. The most preferred form for the oxidising formulation is a lotion.

According to the first aspect, the applying an oxidising formulation to the hair occurs prior to the applying a monomer composition to the hair.

In an embodiment, the method comprises providing the monomer composition to a hair stylist and/or consumer, wherein the hair stylist and/or consumer does not need to pre-mix the monomer composition with a second formulation prior to applying the monomer composition to the hair. The second formulation may be selected from the group consisting of: reducing formulations, pH adjusting formulations, salt formulations, initiator formulations, oxidising formulations, catalyst formulations, and combinations thereof; preferably salt formulations, oxidising formulations, initiator formulations, and mixtures thereof.

In an embodiment, during time x, the hair is exposed to heat. In an embodiment, during time x, the hair is exposed to a relative humidity of at least about 70%, within about 1 hour of applying the monomer composition, and said exposure lasting for about 10 to about 90 min. Means for applying said relative humidity, include, for example: Hairspa ION, by Wella, Darmstadt, Germany; Electronic Master Ionic Action, by Muster; Blitz Super Electronic Ozono, by Ceriotti; Beautivap Digital Ozon and Ozono Energy, by Artem; Mega Ozono, by MediaLine; Mod. 370, by bmp; Steam Machine, by REM; Micro Mist (SD200NIW) and Belmaster (BM-975), by Takara Belmont. A suitable device is described in EP1871194.

The method according to the first aspect may further comprise providing and applying to hair a hair care and/or hair styling composition. Additionally or alternatively it may comprise providing and utilising an implement for applying styling effects to the hair. The hair care and/or hair styling composition may comprise a hair care and/or hair styling active and a cosmetically acceptable carrier - as described herein. Said hair care and/or hair styling active may be selected from the group consisting of hair fixing polymers; conditioning agents, particularly cationic polymers; cleansing agents, particularly surfactants; thickeners; glossing agents; shine-imparting agents; dyes or colour-imparting agents; glitter or coloured particles; silicones; and mixtures thereof. The hair care and/or styling composition may be selected from the group consisting of waxes; gels; hair sprays; mousses; conditioning compositions, particularly leave-in conditioning compositions; finishing sprays; glossing or shine-imparting products; and mixtures thereof. The implement may be selected from the group consisting of blow dryers; flat irons, combs, brushes, curlers, curling tongs, electrical curling devices, foils, scissors, clips, hair bands, and mixtures thereof.

In another embodiment, the present invention relates to a method for chemically modifying the internal region of a hair shaft, wherein the method comprises:

(i) applying an oxidising formulation to the hair wherein the oxidising formulation comprises from about 1% to about 4% hydrogen peroxide, by total weight of the oxidising formulation;
(ii) de-wetting the hair;
(iii) applying a monomer composition to the hair, wherein the monomer composition comprises: an ethylenic monomer having a molecular weight of about 500 g/mole or less; a cosmetically acceptable carrier; an anion; and a cation, wherein the cation is selected from the group consisting of inorganic cations having a charge density of about 0.05 charge/picometre or more.

In another embodiment, the present invention relates to a method for chemically modifying the internal region of a hair shaft, wherein the method comprises:

(i) applying an oxidising formulation to the hair wherein the oxidising formulation comprises: from about 1% to about 4% hydrogen peroxide, by total weight of the oxidizing formulation; an anion; and a cation, wherein the cation is selected from the group consisting of inorganic cations having a charge density of about 0.05 charge/picometre or more;
(ii) de-wetting the hair;
(iii) applying a monomer composition to the hair, wherein the monomer composition comprises; from about 5% to about 14%, by total weight of the monomer composition, of an ethylenic monomer having a molecular weight of about 500 g/mole or less; and a cosmetically acceptable carrier.

In another embodiment, wherein after chemical modifying, the hair exhibits at least one benefit selected from the group consisting of increased style retention, increased style durability, increased appearance of volume, increased resistance to moisture, and combinations thereof.

In another embodiment, wherein after chemical modifying, the hair exhibits increased density of the hair shaft and/or elasticity of the hair shaft.

$^1$H-NMR Analysis

The kinetics of the free radical polymerisation of ethylenic monomer in aqueous media can be followed using $^1$H-NMR. For example, the polymerisation of potassium 3-sulphopropylacrylate is shown in Reaction X below.

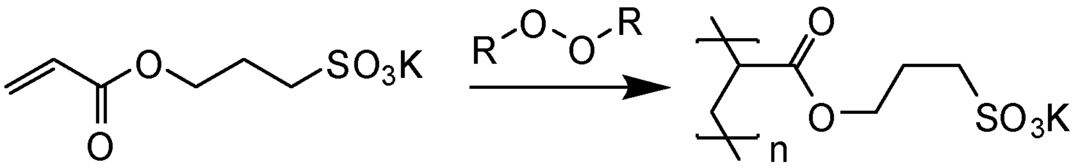

Reaction X

Determination of the conversion from may be performed by comparison of the signals of the residual (unpolymerised) ethylenic monomer to a signal of a reference substance added to the reaction mixture before start of the reaction and/or by comparison to signals stemming from both polymer and ethylenic monomer. Triethylene glycol and sodium benzenesulfonate can be used as inert reference substances. The conversion is deduced by comparison of the integrals. When an inert reference substance is added, the conversion can be calculated according to Equation A:

$$\text{conversion}[\%] = \left(1 - \frac{\dfrac{i_{mono}}{n_{H,mono} \cdot X_{mono}}}{\dfrac{i_{ref}}{n_{H,ref} \cdot X_{ref}}}\right) \cdot 100$$

Equation A

wherein $i_{mono}$: integral of the signal from ethylenic monomer; $n_{H,\,mono}$: number of H atoms in signal from ethylenic monomer; $X_{mono}$: amount of ethylenic monomer before start of the reaction in moles; $i_{ref}$: integral of the signal from reference; $n_{H,\,ref}$: number of H atoms in signal from reference; $X_{ref}$: amount of reference in moles.

For calculation of the conversion by comparison of residual ethylenic monomer signals and combined ethylenic monomer and polymer signals, Equation B can be utilised:

$$\text{conversion}[\%] = \left(1 - \frac{\dfrac{i_{mono}}{n_{H,mono}}}{\dfrac{i_{comb}}{n_{H,comb}}}\right) \cdot 100$$

Equation B

wherein $i_{mono}$: integral of the signal from the ethylenic monomer; $n_{H,\,mono}$: number of H atoms in signal from ethylenic monomer; $i_{comb}$: integral of the combined signal from ethylenic monomer and polymer; $n_{H,\,comb}$: number of H atoms in combined signal from ethylenic monomer and polymer.

The following experimental procedure may be employed: ethylenic monomer and additives (when appropriate) in a round flask fitted with a silicone stopper are dissolved in 18 ml of the appropriate solvent (buffer with or without addition of salt, or ultra-pure demineralised water). The buffer that can be utilised for experiments at pH 5.8 is prepared as follows: 119.2 mg of $NaH_2PO_4$ and 141.9 mg of $Na_2HPO_4$ is dissolved in 110 ml of ultra pure water, then the pH is

adjusted to the final value of 5.8 by addition of diluted phosphoric acid. The desired amount of initiator is dissolved in some ml of the same solvent (exact amount of solvent is adjusted individually to bring the total weight of the reaction mixture to 25.0 g). The reaction temperature is adjusted by an oil bath with contact thermometer. The reaction is started by transfer of the initiator solution to the reaction mixture with a syringe. In case no initiator is used, the flask with the starting materials is kept outside the oil bath before the reaction and the moment of contact with the oil bath is regarded as the starting point. At defined points in time, samples of 3.0 ml are drawn with a syringe. To stop the reaction, these samples are immediately exposed to air and 0.100 g of hydroquinone is added. The samples are freeze-dried separately and analysed by $^{1}$H-NMR spectroscopy for the determination of the conversion.

As an example, the structure of 3-sulfopropyl acrylate potassium salt is indicated below in Formula I, wherein the $H_1$ indicates the position of the hydrogen atom that can be analysed by NMR

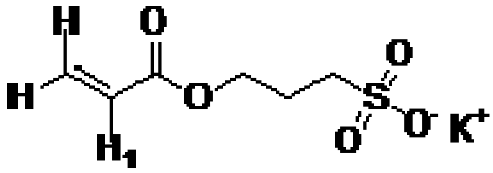

Formula I

Signals at 6.5 to 6.4 ppm (parts per million) corresponds to $H_1$ atom in the residual ethylenic monomer. Signals at 3.8 to 3.6 ppm corresponds to 12 H atoms in triethylene glycol. A signal at 4.4 to 4.2 ppm corresponds to the combined signal.

Utilising this technique, Fig 1 demonstrates the conversion of ethylenic monomer species into polymer species. The y axis labelled "C" shows conversion in percentage by mole of ethylenic monomer and the x-axis, which is labeled 't', shows time in mins. In the experiment labelled "Sr", the monomer composition comprised 5.0% (by weight) $SrCl_2$. In the experiment labelled "Al", the monomer composition comprised 2.4% (by weight) $Al_2(SO_4)_3$ x $18(H_2O)$ i.e. aluminium sulfate octadecahydrate. Both experiments were carried out using 2.00 g 3-sulfopropyl acrylate as ethylenic monomer, in the presence of oxygen, at pH 5.8, at 20°C, utilising 1.0% (by weight) $Na_2S_2O_8$ as initiator, 127 mg cysteine hydrochloride at a 1:1 molar ratio with the initiator, wherein $C_6H_5SO_3Na$ was utilized as a reference substance, and in the absence of hair.

Conclusions drawn from Fig 1 include that polymerisation occurs such that almost all ethylenic monomer is converted and that there is only a subtle difference in reaction kinetics when compositions comprising aluminium and strontium cations are compared.

GPC Analysis

The molecular weights of polymer resultant from the polymerisation of ethylenic monomers may be obtained by routine GPC in water-based (anionic) solution. The GPC equipment comprises a single-channel degassing system [WGE-Dr.Bures], an isocratic pump [P 1000 of Spectra Physics], GPC-liquid 0.1 mol/l $NaNO_3$ at a flux rate of 1.0 ml/min, auto-sampler AS 1000 of Spectra Physics, sampling 100μl, PSS SUPREMA column (1) Guard (8 x 50 mm), (2) Suprema 3000 A (8 x 300 mm; 10μm), (3) Suprema 1000 A (8 x 300 mm; 10μm), (4) Suprema 100 A (8 x 300 mm; 10μm), column-oven K-7 of TECHLAB GmbH, Germany [at T=30°C], a UV -Detector UV 2000 of Spectra Physics, an RI-Detector Refractometer SEC-2010 of WGE Dr. Bures, Germany. Calibration was performed with Pullulan-Standards over a molecular weight range of 0.3 to 710 kDa. Data analysis was done using software WinGPC Unity by PSS.

The GPC data is presented in Fig 2. The x-axis represented by the letter 'a' is the molecular weight in Daltons (Da). The y-axis represented by the letter 'b' is the intensity. I = as per the invention (dash marker); C = comparative i.e. not per the invention (cross marker). It is known in the art that the resulting polymer molecular weight is inversely proportional to the square root of the initial radical concentration - a radical in this sense being a ethylenic monomer having been activated by an initiator. Thus, it is important to understand the kinetics of ethylenic monomer consumption in order to make reliable conclusions from the GPC data. Hence, the applicant has utilised $^{1}$H-NMR to follow the kinetics of ethylenic monomer conversion. Conclusions drawn from Fig 2 thus include that a higher molecular weight polymer results for a monomer composition comprising aluminium cations versus for a monomer composition comprising strontium cations.

$^{14}$C-Experiments: Part I

The aim of this study was to study the polymerisation reaction of 3-sulfopropylacrylate (3-SPA) that takes place inside hair. We used a $^{14}$C- radio-labelled version (labelled C-atom: ★) of 3-SPA:

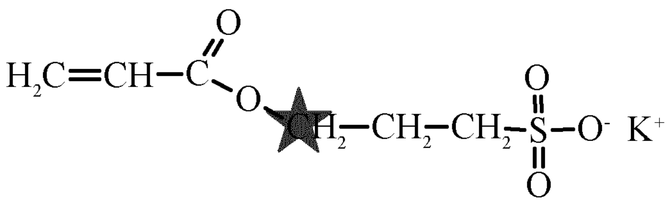

Procedure: Bundles of exactly 20 hair fibres are double bleached. The bundles are treated with either normal 3-SPA or the $^{14}$C-labelled version. The kind of treatment is according to the method: (1) 10 min pre-treatment formulation (2% $H_2O_2$); (2) "Towel" drying (paper tissue); (3) 30 min treatment with 3-SPA in water or in hair treatment formulation; (4) optionally rinsing with water. The exact procedure for each particular hair bundle was done as follows:

| Sample | 10 min pre-treatment | Test Preparation | Rinse | Exposur e Time |
|---|---|---|---|---|
| Bundle A | Yes | Blank: Non labelled 3-SPA in water | No | 30 min |
| Bundle B<br>Bundle C | Yes<br>Yes | $^{14}$C-3-SPA in water<br>$^{14}$C-3-SPA in water | No<br>Yes | 30 min<br>30 min |
| Bundle D<br>Bundle E | Yes<br>Yes | $^{14}$C-3-SPA in treatment formulation<br>$^{14}$C-3-SPA in treatment formulation | No<br>Yes | 30 min<br>30 min |

The detection of the $^{14}$C-3-SPA in the hair strands was done by micro-autoradiography. The hair strands were cut to 1.5 cm lengths and the cross-section of the strands were exposed to a silver halide photographic emulsion for 4 weeks in a dark room. The photographic film was developed and photographs thereof were taken using a microscope. Beta particles convert the silver halide into metallic silver, which is observable as black dots. The results are shown in the below table and in Fig. 3.

| Hair Bundle | Treatment | Average Ag-Grain Intensity | Silver Grain Distribution | Representative Photomicrograph (x 400 magnification) |
|---|---|---|---|---|
| A (Blank) | - 3-SPA (non-labelled) in water<br>- No rinse | ++ | Cuticle and cortex | Fig. 3A |
| B | - $^{14}$C-labelled 3-SPA in water<br>- No rinse | +++ | Cuticle and cortex | Fig. 3B |
| C | - $^{14}$C-labelled 3-SPA in water<br>- Rinsed | +++ | Cuticle and cortex | Fig. 3C |
| D | - $^{14}$C-labelled 3-SPA in formulation<br>- No rinse | ++++ | Cuticle and cortex | Fig. 3D |
| E | - $^{14}$C-labelled 3-SPA in formulation<br>- Rinsed | +++ | Cuticle and cortex | Fig. 3E |

Interpretation and discussion: All hair strands which had been treated with labelled 3-SPA, showed clearly that radioactive material has permeated not only the hair cuticle but also considerable parts of the cortex. A mostly widely homogeneous (circular) distribution is also seen. What is even more conclusive than the high concentration of silver grains, is their accumulation at the outer half of the diffusion-passage. All pictures show a preference for accumulation in the outer part of the cortex, but for Bundle D there is even a kind of border line in the inner cortex area visible. This gives a strong hint that the silver particles really represent penetration behaviour of the 3-SPA monomer and not an artefact, for instance due to the cutting process. From the above pictures in Fig 3, it is not so apparent that rinsing has no influence on the result. However, expert opinion of this technique was that the rinsing step had only low impact on

the radioactivity inside the hair. This is another hint that the treatment according to the present invention is not merely a surface effect.

$^{14}$C-Experiments: Part II

Procedure: As per Part I, except the pre-treatment according to (1) may be optionally water; the treatment time (step [3]) with the monomer composition is increased to 24 h; and the rinsing (4) is always performed:

The exact procedure for each particular hair bundle has been done as follows:

| Sample | $H_2O_2$ Pre-treatment | Test Preparation | Rinsing | Exposure Time |
|---|---|---|---|---|
| A | Yes | $^{14}$C-3-SPA in water | Yes | 24 h |
| B | No | $^{14}$C-3-SPA in water | Yes | 24 h |
| C | Yes | $^{14}$C-3-SPA in formulation | Yes | 24 h |
| D | No | $^{14}$C-3-SPA in formulation | Yes | 24 h |

The detection of the $^{14}$C-3-SPA in the hair strands was done by micro-autoradiography. The results are shown in the below table and in Fig. 4.

| Hair Bundle | Pre-Treatment | Average Ag-Grain Intensity | Silver Grain Distribution | Representative Photomicrograph (x 400 magnification) |
|---|---|---|---|---|
| A | 10 min 2% $H_2O_2$ | ++ | Cuticle and cortex | Fig. 4A |
| B | 10 min water | + | Cortex only | Fig. 4B |
| C | 10 min 2% $H_2O_2$ | ++ | Cuticle and cortex | Fig. 4C |
| D | 10 min water | +++ | Cortex only | Fig. 4D |

Interpretation and discussion: experiments showed in all cases of $H_2O_2$ pre-treatment radioactivity both, in the cuticle and in the cortex, whereas in case of pre-treating only with water, no radioactivity could be found in the cuticle, but still in the cortex. Other in-vitro-experiments showed that not only the monomer, but also the poly-3-SPA is water soluble. However, dissolving polymer, once it had been dried, takes considerably longer (some hours) than dissolving dry monomer (some seconds). These data thus suggest that the differences in wash resistance are due to polymerisation level. All the 20 hair fibres of each sample without $H_2O_2$ pre-treatment lost their radioactivity of the cuticle area by washing, whereas all the other samples kept it.

Hair density data

The amount of "add-on" i.e. the percentage change in weight of the hair following treatment is measured. Before and after treatment, the switches are weighed on an analytical balance and the difference calculated as a percent. For both weighings, the hair is in dry form. Twenty 2.5 cm switches are permed and 2 times bleached before treatment. An oxidizing formulation comprising 2% $H_2O_2$ is then applied for 5 min. Then a monomer composition is then applied for 30 min at 45°C, wherein the monomer composition comprises 12% 3-SPA. The switches are then washed with neutral shampoo and then a finishing formulation comprising with 2% $H_2O_2$ is then applied. The switches were air dried and weighed. The result of this add-on experiment showed an average weight increase of 2.93%.

The diameter of individual hair fibres is also measured with a laser micrometer. 25 single hair fibres from the same batch of European hair are taken and their diameters measured before and after treatment according to the present invention. The fibres are bleached twice before treatment. An oxidizing formulation comprising 2% $H_2O_2$ is applied, a monomer composition is then applied (comprising 12% 3-SPA), and a finishing formulation comprising with 2% $H_2O_2$ is applied. For each fibre, diameters were measured at a plurality of points along the fibre length and such diameter measurements are averaged for each fibre - an average minimum diameter of about 60 micron and average maximum diameter of about 86 micron was measured. Furthermore, the average across the 25 fibres is also calculated. The result of these experiments was a very slight decrease in average hair diameter versus untreated hair fibres - the average

diameter of the untreated fibres was 73.2 micron (standard deviation 16.7 micron) and the average diameter of the treated fibres is 73.0 micron (standard deviation 16.6 micron).

Interpretation and discussion: Since there is an increased in add-on and a slight decrease in hair diameter, it is concluded that the density of the hair fibres increased.

CONSUMER AND STYLIST DATA

The present invention is tested on consumers. Consumers are selected according to their needs. Consumer segment 1 comprises consumers desiring fuller, more voluminous and thicker hair because their hair is currently limp and thin. Consumer segment 2 comprises consumers desiring hair with a tamed and more defined appearance because their hair is currently thick and unruly.

In the test, each consumer receives a treatment pursuant to the present invention on one half of their head of hair, and a treatment not pursuant to the present invention on the other half. In all cases, the treatment is applied to wet hair. At the end of the test, the two halves are compared by gathering both consumer and stylist feedback. The methods that may be applied to each consumer half head are shown in the table below entitled Table II.

Table II

| Steps | Comparative Method | Inventive Method 1 | Inventive Method 2 |
|---|---|---|---|
| Step 1 | Application of a placebo treatment. Treatment time y = 5 min. | Application of 2% (w/w) $H_2O_2$ lotion. Treatment time y = 5 min. | Application of 2% (w/w) $H_2O_2$ lotion. Treatment time y = 5 min. |
| Step 2 | Application of monomer composition, which comprises 8% 3-sulfopropylacrylate, $Al_2(SO_4)_3$, which is premixed with sodium persulfate. Treatment time x = 30 min. | Application of monomer composition, which comprises 8% 3-sulfopropylacrylate, $Al_2(SO_4)_3$. Treatment time x = 30 min. | Application of monomer composition, which comprises 12% 3-sulfopropylacrylate, $Al_2(SO_4)_3$. Treatment time x = 30 min. |
| Step 3 | Application of oxidizing formulation comprising 2% (w/w) $H_2O_2$. | Application of oxidizing formulation comprising 2% (w/w) $H_2O_2$. | Application of oxidizing formulation comprising 2% (w/w) $H_2O_2$ |

In this study, a total of 47 consumers received the comparative method, of which 26 were from consumer segment 1 and 21 from consumer segment 2. A total of 20 consumers received inventive method 1, of which 10 were from consumer segment 1 and 10 from consumer segment 2. A total of 27 consumers received inventive method 2, of which 16 were from consumer segment 1 and 11 from consumer segment 2.

Immediately following their salon visit where the above methods are applied, the consumers are asked a variety of questions about their hair, for example "Does your hair feel full?". For each question, the consumer must choose one of five discrete answers: Completely agree, mostly agree, neither agree nor disagree, mostly disagree, completely disagree. For these questions, the percentage of consumers that answer with either 'completely agree' or 'mostly agree' for each half head is calculated. A further question is asked, namely whether the benefits of the treatment are what they expected. For this question, a five-point scale was also used and the percentage of consumer answering either 'exceeded expectations', 'fully met expectations', or 'mostly met expectations' is calculated. The results of the above-mentioned questions answered immediately after their salon visit can be found in Table III below.

Table III

| Consumer Segment 1 | Comparative Method | Inventive Method 2 | Consumer Segment 2 | Comparative Method | Inventive Method 2 |
|---|---|---|---|---|---|
| meets/exceeds expectations | 68% | 88% | meets/exceeds expectations | 70% | 100% |
| Full | 68% | 94% | smooth | 87% | 100% |
| Smooth | 64% | 88% | shiny | 60% | 92% |
| Healthy | 59% | 75% | healthy | 60% | 83% |
| Strong | 64% | 75% | tamed | 53% | 83% |

Four weeks after treatment the same consumers are asked similar questions. The results of the above-mentioned questions four weeks after treatment can be found in Table IV below.

Table IV

| Consumer Segment 1 | Comparative Method | Inventive Method 2 | Consumer Segment 2 | Comparative Method | Inventive Method 2 |
|---|---|---|---|---|---|
| meets/exceeds expectations | 59% | 88% | meets/exceeds expectations | 59% | 83% |
| easy to style | 77% | 94% | easy to style | 67% | 83% |
| Smooth | 86% | 94% | smooth | 67% | 75% |
| Hold | 64% | 94% | healthy | 60% | 67% |
| Full | 64% | 81% | moisturized | 60% | 58% |

During the four week period after treatment, each consumer is asked to keep a hair diary. Each day the consumer must state whether they notice a difference in their hair versus how their hair was prior to the treatment. Also, each day they had to note down more specifically how their hair felt e.g. shiny, voluminous etc. Using these data, the percentage of consumers that after a certain number of washes still see a benefit from the treatment is calculated. These data are shown in Table V below.

Table V

| Number of washes | <5 | 5-9 | 10-14 | 15-19 | 20-29 | ≥29 | ≥15 |
|---|---|---|---|---|---|---|---|
| Comparative Method | 25% | 18% | 7% | 25% | 11% | 14% | 50% |
| Inventive Method 2 | 14% | 21% | 4% | 29% | 14% | 18% | 61% |

Furthermore, an overall analysis of the consumer feedback was carried out. Immediately after treatment and four weeks after treatment, consumers are also asked: How would you rate the treatment overall? They must answer this with one of the following options: excellent, very good, good, not so good, poor. The number of consumers answering this question 'excellent' or 'very good', is used to calculate a percentage for the "overall rating". These data, in addition to a summary of the responses of consumers in relation to whether the treatment met their expectations, can be found in Table VI below.

Table VI

| Rating | Method / Time | Comparative Method (n=40) | Inventive Method 1 (n=20) | Inventive Method 2 (n=29) |
|---|---|---|---|---|
| Meets/exceeds expectations [%] | Immediately after treatment | 69 | 95 | 93 |
| | Four weeks after treatment | 56 | 75 | 83 |
| Overall rating [%] | Immediately after treatment | 46 | 65 | 71 |
| | Four weeks after treatment | 44 | 65 | 62 |

Moreover, stylists are asked for their opinion as to the efficacy of the treatment on the consumers. Each stylist is asked to respond to each criterion using a seven point scale wherein the stylist can provide a rating of one of the following options: +3, +2, +1, 0, -1, -2, -3. The results of this study are shown below in Table VII.

Table VII

| Half side having received an inventive method vs. half side having received the comparative method | | Inventive Method 1 (n=20) | Inventive Method 2 (n=31) |
|---|---|---|---|
| Immediately | Wet feel | 1.5* | 1.3* |
| | Wet combability | 1.3* | 1.4* |
| | Wet heaviness | -0.8^ | -0.7^ |
| | Wet elasticity | 0.7* | 0.1$^{=}$ |
| | Dry volume | 0.3$^{=}$ | 0.3$^{=}$ |
| | Firmness / stability | 0.0$^{=}$ | 0.1$^{=}$ |
| | Curl definition or structure | 0.4$^{=}$ | 0.3$^{=}$ |
| | Dry feel | 1.1* | 1.2* |
| after treatment | Dry combability | 1.0* | 0.4$^{=}$ |
| | Dry elasticity | 0.7* | 0.5$^{=}$ |
| | Dry heaviness | -0.3$^{=}$ | -0.6^ |
| | Anti-frizz | 1.1* | 0.8* |
| | Static | 1.2* | 0.9* |
| | Ease of brushing | 0.2$^{=}$ | 0.2$^{=}$ |
| | Creation of style | 0.7* | 0.5$^{=}$ |
| | Drying time | 0.1$^{=}$ | 0.0$^{=}$ |
| | Ease of styling | 0.6* | 0.5$^{=}$ |
| | Shine | 0.4$^{=}$ | 0.3$^{=}$ |
| | Colour loss | 0.0$^{=}$ | 0.0$^{=}$ |
| After 4 weeks | Colour loss | 0.0$^{=}$ | 0.1$^{=}$ |
| | Wet feel | 0.9* | 1.0* |
| | Wet combability | 0.7* | 0.9* |
| | Dry feel | 0.9* | 0.9* |
| | Volume | 0.6* | 0.1$^{=}$ |
| | Shine | 0.0$^{=}$ | 0.3$^{=}$ |
| Key: * = results show inventive method is significantly better than comparative method; ^ = results show inventive method is significantly worse than comparative method; $^{=}$ = results show inventive method is the same as comparative method. | | | |

A conclusion drawn from these consumer and stylist data includes that the method pursuant to the present invention results in an improved performance versus the comparative method not pursuant to the present invention.

EXAMPLES

Oxidising Formulation: Examples 1 - 3

| | Ex. 1 | Ex. 2 | Ex. 3 |
|---|---|---|---|
| **Phase 1** | | | |
| Purified water | 48.00 | 48.00 | 48.00 |
| Disodium phosphate | 0.08 | 0.08 | 0.08 |
| Salicylic acid | 0.10 | 0.10 | 0.10 |
| **Phase 2** | | | |
| Purified water | 49.96 | 47.35 | 49.56 |
| Hydrogen peroxide | 1.80 | 2.00 | 2.20 |
| Aluminium sulfate octadecahydrate | - | 2.40 | - |
| Phosphoric acid | 0.06 | 0.07 | 0.06 |
| Total | 100.00 | 100.00 | 100.00 |

Phase 1 and phase 2 are created separately and then mixed together. The phase 1 components are mixed together and heated to 80°C until the salicylic acid is dissolved. Phase 1 is then stirred and cooled to 40°C. Then phase 2 is created and mixed in with phase 1 with stirring for 5 min.

Following application of the oxidising formulation to the hair, the hair is de-wetted by towel drying the hair. Then, a monomer composition is applied to the hair.

Monomer Composition: Examples 1 - 4

| | Ex. 1 | Ex. 2 | Ex. 3 | Ex. 4 |
|---|---|---|---|---|
| **Phase 1** | | | | |
| Purified water | 66.90 | 66.90 | 64.90 | 64.90 |
| Hydroxyethylcellulose [1] | 0.20 | 0.20 | 0.20 | 0.20 |
| Disodium EDTA | 0.12 | 0.12 | 0.12 | 0.12 |
| Xanthan gum [2] | 0.50 | 0.50 | 0.50 | 0.50 |
| Aluminium sulfate octadecahydrate | 2.40 | 2.40 | 2.40 | 2.40 |
| **Phase 2** | | | | |
| Purified water | 20.22 | 18.22 | 16.22 | 13.22 |
| Sodium hydroxide | 0.76 | 0.76 | 0.76 | 0.76 |
| **Phase 3** | | | | |
| 2-phenoxyethanol | 1.00 | 1.00 | 1.00 | 1.00 |
| Methyl parabene | 0.20 | 0.20 | 0.20 | 0.20 |
| Coceth-10 [3] | 0.70 | 0.70 | 0.70 | 0.70 |
| PEG-35 castor oil [4] | 0.70 | 0.70 | 0.70 | 0.70 |
| Fragrance | 0.30 | 0.30 | 0.30 | 0.30 |
| 3-sulfopropyl acrylate potassium salt [5] | 6.00 | 8.00 | 12.00 | 15.00 |
| Total | 100.00 | 100.00 | 100.00 | 100.00 |
| **Key: [1] =** Cellosize® HEC QP-4400H from Dow Europe GmbH; [2] = Keltrol® CG-T from CP Kelco A HUBER COMPANY; [3] = Genapol® C-100 from Clariant Produkte GmbH (Deutschland); [4] = Cremophor® EL from BASF The Chemical Company; [5] = 3-sulfopropyl acrylate potassium salt from Raschig. | | | | |

Phase 1 and phase 2 are created separately and then mixed together. Phase 3 without the 3-sulfopropyl acrylate potassium salt is formed with heating to 40°C, and then mixed with the other two phases. Finally the salt is added and the final monomer composition stirred for 10 mins.

Monomer Composition: Examples 5 - 8

| | Ex. 5 | Ex. 6 | Ex. 7 | Ex. 8 |
|---|---|---|---|---|
| **Phase 1** | | | | |
| Purified water | 77.98 | 79.98 | 81.98 | 83.98 |
| Hydroxyethylcellulose [6] | 0.70 | 0.70 | 0.70 | 0.70 |
| Disodium EDTA | 0.12 | 0.12 | 0.12 | 0.12 |
| Formic acid | 0.30 | 0.30 | 0.30 | 0.30 |
| **Phase 2** | | | | |
| 2-phenoxyethanol | 1.00 | 1.00 | 1.00 | 1.00 |
| p-hydroxybenzoic acid methylester | 0.20 | 0.20 | 0.20 | 0.20 |
| Coceth-10 [3] | 0.70 | 0.70 | 0.70 | 0.70 |
| PEG-35 castor oil [4] | 0.70 | 0.70 | 0.70 | 0.70 |
| Fragrance | 0.30 | 0.30 | 0.30 | 0.30 |
| **Phase 3** | | | | |
| 3-sulfopropyl acrylate potassium salt [5] | 6.00 | 8.00 | 10.00 | 12.00 |
| Total | 100.00 | 100.00 | 100.0 0 | 100.00 |
| **Key:** as above, and [6] = Natrosol® 250 HHR from Herkules. | | | | |

Phase 1 and Phase 2 are created separately. Phase 1, but with the exception of the formic acid, is created with stirring for 2 min, followed by heating to 50°C with stirring until the hydroxyethylcellulose is swollen. After cooling the solution down to less than 30°C, the formic acid is added until pH of Phase 1 reaches pH 2.5. To create Phase 2, first the 2-phenoxyethanol and the p-hydroxybenzoic acid methylester are mixed until a solution is formed. Then the 40°C coceth-10 is added to the solution and stirred. Final Phase 2 is created by subsequently mixing in the PEG-35 castor oil and the fragrance. Then Phase 2 is added to Phase 1, and then the 3-sulfopropyl acrylate potassium salt is added and the resulting monomer composition mixed for 5 min.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** Method for chemically modifying the internal region of a hair shaft, wherein the method comprises:

(i) applying an oxidising formulation to the hair, wherein the oxidising formulation comprises an oxidising agent, and wherein the oxidising agent is selected from the group consisting of: peroxides, preferably hydrogen peroxide; persulfates, preferably potassium persulfate or sodium persulfate; and mixtures thereof; followed by
(ii) de-wetting the hair; followed by
(iii) applying a monomer composition to the hair, wherein the monomer composition comprises an ethylenic monomer having a molecular weight of 500 g/mole or less and a cosmetically acceptable carrier;

wherein the de-wetting the hair comprises the application of an absorbent material to the hair such that wetness is

transferred from the hair to the absorbent material and wherein
the wetness comprises the cosmetically acceptable carrier;
and wherein the de-wetting the hair does not comprise rinsing the oxidising formulation from the hair.

**2.** The method according to claim 1, further comprising one or more of the following:

- allowing the monomer composition to remain on the hair for a period of time x, wherein the time x is from 1 min to 120 mins;
- rinsing the hair;
- washing the hair.

**3.** The method according to any of the preceding claims, the method comprises providing the monomer composition to a hair stylist and/or consumer, wherein the hair stylist and/or consumer does not need to pre-mix the monomer composition with a second formulation prior to applying the monomer composition to the hair.

**4.** The method according to any of the preceding claims, wherein the ethylenic monomer is selected from the group consisting of: mesaconic acid, 2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2- methylenebutyrate, methacryloyl-L-lysine, N-(2-hydroxypropyl)methacrylamide, 2- acrylamidodiglycolic acid, 2-ethoxyethyl acrylate, 2-butoxyethyl acrylate, N-isopropylmethacryalmide, 2-aminoethyl methacrylate, 2-bromoethyl acrylate, 3-(dimethylamino)propyl acrylate, (3-acrylamidopropyl)trimethyl ammonium salt, [2-(acryloyloxy)ethyl]-trimethylammonium salt, alkylacetamidoacrylate, sulfoalkyl(meth)acrylate, 3-sulfopropyl acrylate, 3-sulfopropyl methacrylate, and salts, isomers, derivatives, and mixtures thereof.

**5.** The method according to any of claims 2 to 4, wherein the time x is from 5 mins to 100 mins, more preferably from 10 mins to 90 mins, most preferably from 20 to 60 mins.

**6.** The method according to any of the preceding claims, wherein after applying the oxidising formulation to the hair but prior to de-wetting the hair, the oxidizing formulation is allowed to remain on the hair for a period of time y, wherein time y is from 1 min to 120 mins, preferably from 2 mins to 45 mins, more preferably from 3 mins to 20 mins, most preferably from 4 mins to 10 mins.

**7.** The method according to any of the preceding claims, wherein the monomer composition and/or the oxidising formulation further comprises a cation and an anion; wherein the cation is selected from the group consisting of inorganic cations having a charge density of 0.05 charge/picometre or more.

**8.** The method according to any of the preceding claims, wherein the monomer composition further comprises a viscosity-increasing means.

**9.** The method according to claim 8, wherein the viscosity-increasing means is a viscosity-increasing agent selected from the group consisting of non-ionic thickeners, anionic thickeners, cationic thickeners, amphoteric thickeners, and mixtures thereof.

**10.** The method according to any of claims 8 to 9, wherein the viscosity-increasing agent comprises at least one polysaccharide, preferably at least one heteropolysaccharide.

**11.** The method according to any of claims 2 to 10 wherein during time x, the hair is exposed to a relative humidity of at least 70%, within 1 hour of applying the monomer composition, and said exposure lasting for 10 to 90 min.

### Patentansprüche

**1.** Verfahren zur chemischen Modifizierung des Innenbereichs eines Haarschafts, wobei das Verfahren Folgendes umfasst:

(i) Auftragen einer oxidierenden Formulierung auf das Haar, wobei die oxidierende Formulierung ein Oxidationsmittel umfasst und wobei das Oxidationsmittel ausgewählt ist aus der Gruppe bestehend aus: Peroxiden, vorzugsweise Wasserstoffperoxid; Persulfaten, vorzugsweise Kaliumpersulfat oder Natriumpersulfat; und Mischungen davon; gefolgt von
(ii) Entfeuchten des Haars; gefolgt von
(iii) Auftragen einer Monomerzusammensetzung auf das Haar, wobei die Monomerzusammensetzung ein ethylenisches Monomer mit einem Molekulargewicht von 500 g/mol oder weniger und einen kosmetisch verträglichen Träger umfasst;

wobei das Entfeuchten des Haars das Auftragen eines Absorptionsmaterials auf das Haar umfasst, so dass die Feuchtigkeit von dem Haar auf den Absorptionsmaterial übertragen wird, und wobei die Feuchtigkeit den kosmetisch akzeptablen Träger umfasst; und wobei das Entfeuchten des Haars nicht das Ausspülen der oxidierenden Formulierung aus dem Haar umfasst.

**2.** Verfahren nach Anspruch 1, ferner umfassend eines oder mehrere von Folgendem:

Belassen der Monomerzusammensetzung auf dem Haar für einen Zeitraum x, wobei die Zeit x von 1 Min. bis 120 Min. beträgt;
Ausspülen des Haars;
Waschen des Haars.

**3.** Verfahren nach einem der vorstehenden Ansprüche, wobei das Verfahren das Bereitstellen der Monomerzusammensetzung für einen Friseur und/oder Verbraucher umfasst, wobei der Friseur und/oder Verbraucher die Monomerzusammensetzung vor dem Auftragen der Monomerzusammensetzung auf das Haar nicht mit einer zweiten Formulierung vormischen muss.

**4.** Verfahren nach einem der vorstehenden Ansprüche, wobei das ethylenische Monomer ausgewählt ist aus der Gruppe bestehend aus: Mesaconsäure, 2-Pentensäure, Tiglinsäure, Tiglinsäureestern, Furan-3-acrylsäure, 2-Acrylamido-2-methyl-1-propansulfonsäure, Maleaminsäure, 3-Aminocrotonsäure, Crotonsäureestern, Itaconsäureanhydrid, Trimethylsilylacrylat, Poly(ethylenglycol)acrylaten, N-Vinylacetamid, 2-Acetamidoacrylsäure, Vinylsulfonsäure, Tetrahydrofurfurylacrylat, N-Methyl-N-vinylacetamid, Vinylpropionat, Vinylanisol, Vinylcrotonat, Methyl-3-hydroxy-2-methylenbutyrat, Methacryloyl-L-lysin, N-(2-Hydroxypropyl)methacrylamid, 2-Acrylamidodiglycolsäure, 2-Ethoxyethylacrylat, 2-Butoxyethylacrylat, N-Isopropylmethacrylamid, 2-Aminoethylmethacrylat, 2-Bromethylacrylat, 3-(Dimethylamino)propylacrylat, (3-Acrylamidopropyl)trimethylammoniumsalz, [2-(Acryloyloxy)ethyl]trimethylammoniumsalz, Alkylacetamidoacrylat, Sulfoalkyl(meth)acrylat, 3-Sulfopropylacrylat, 3-Sulfopropylmethacrylat und Salzen, Isomeren, Derivaten und Mischungen davon.

**5.** Verfahren nach einem der Ansprüche 2 bis 4, wobei die Zeit x von 5 Min. bis 100 Min., mehr bevorzugt von 10 Min. bis 90 Min., am meisten bevorzugt von 20 bis 60 Min. beträgt.

**6.** Verfahren nach einem der vorstehenden Ansprüche, wobei nach dem Auftragen der oxidierenden Formulierung auf das Haar, jedoch vor dem Entfeuchten des Haars, die oxidierende Formulierung für einen Zeitraum y auf dem Haar belassen wird, wobei die Zeit y von 1 Min. bis 120 Min., vorzugsweise von 2 Min. bis 45 Min., mehr bevorzugt von 3 Min. bis 20 Min., am meisten bevorzugt von 4 Min. bis 10 Min. beträgt.

**7.** Verfahren nach einem der vorstehenden Ansprüche, wobei die Monomerzusammensetzung und/oder die oxidierende Formulierung ferner ein Kation und ein Anion umfassen; wobei das Kation ausgewählt ist aus der Gruppe bestehend aus anorganischen Kationen mit einer Ladungsdichte von 0,05 Ladungen/Pikometer oder mehr.

**8.** Verfahren nach einem der vorstehenden Ansprüche, wobei die Monomerzusammensetzung ferner ein viskositätssteigerndes Mittel umfasst.

**9.** Verfahren nach Anspruch 8, wobei das viskositätssteigernde Mittel ein viskositätssteigerndes Mittel ist, das ausgewählt ist aus der Gruppe bestehend aus nichtionischen Verdickungsmitteln, anionischen Verdickungsmitteln, kationischen Verdickungsmitteln, amphoteren Verdickungsmitteln und Mischungen davon.

**10.** Verfahren nach einem der Ansprüche 8 bis 9, wobei das viskositätssteigernde Mittel mindestens ein Polysaccharid, vorzugsweise mindestens ein Heteropolysaccharid umfasst.

**11.** Verfahren nach einem der Ansprüche 2 bis 10, wobei während der Zeit x das Haar einer relativen Feuchtigkeit von mindestens 70 % innerhalb von 1 Stunde nach Auftragen der Monomerzusammensetzung ausgesetzt wird und das Aussetzen 10 bis 90 Min. andauert.

## Revendications

**1.** Procédé pour modifier chimiquement la région interne d'une tige capillaire, où le procédé comprend :

(i) l'application d'une formulation oxydante sur les cheveux, dans lequel la formulation oxydante comprend un agent oxydant, et dans lequel l'agent oxydant est choisi dans le groupe constitué de : peroxydes, de préférence le peroxyde d'hydrogène ; persulfates, de préférence le persulfate de potassium ou le persulfate de sodium ; et des mélanges de ceux-ci ; suivie par ;
(ii) un démouillage des cheveux ;
(iii) l'application d'une composition monomère sur les cheveux, dans lequel la composition monomère comprend un monomère éthylénique ayant une masse moléculaire de 500 g/mole ou moins et un véhicule acceptable sur le plan cosmétique.

dans lequel le démouillage des cheveux comprend l'application d'un matériau absorbant sur les cheveux de telle sorte que l'humidité est transférée des cheveux sur le matériau absorbant et dans lequel l'humidité comprend le véhicule acceptable sur le plan cosmétique ;

**2.** Procédé selon la revendication 1, comprenant en outre une ou plusieurs des étapes suivantes :

- laisser la composition monomère rester sur les cheveux pendant un laps de temps x, dans lequel le temps x va de 1 min à 120 min ;
- rincer les cheveux ;
- laver les cheveux.

**3.** Procédé selon l'une quelconque des revendications précédentes, le procédé comprenant la fourniture de la composition monomère à un coiffeur et/ou à un consommateur, dans lequel le coiffeur et/ou le consommateur n'ont pas besoin de prémélanger la composition monomère avec une deuxième formulation avant l'application de la composition monomère sur les cheveux.

**4.** Procédé selon l'une quelconque des revendications précédentes, dans lequel le monomère éthylénique est choisi dans le groupe constitué de : acide mésaconique, acide 2-penténoïque, acide tiglique, esters d'acide tiglique, acide furan-3-acrylique, acide 2-acrylamido-2- méthyl-1-propane sulfonique, acide maléamique, acide 3-aminocrotonique, esters d'acide crotonique, anhydride itaconique, acrylate de triméthylsilyle, acrylates de poly(éthylèneglycol), N-vinylacétamide, acide 2-acétamidoacrylique, acide vinylsulfonique, acrylate de tétrahydrofurfuryle, N-méthyl-N-vinylacétamide, propionate de vinyle, vinylanisole, crotonate de vinyle, 3-hydroxy-2-méthylènebutyrate de méthyle, méthacryloyl-L-lysine, N-(2-hydroxypropyl)méthacrylamide, acide 2-acrylamidodiglycolique, acrylate de 2-éthoxyéthyle, acrylate de 2-butoxyéthyle, N-isopropylméthacrylamide, méthacrylate de 2-aminoéthyle, acrylate de 2-bromoéthyle, acrylate de 3-(diméthylamino)propyle, sel de (3-acrylamidopropyl)triméthylammonium, sel de [2-(acryloyloxy)éthyl]-triméthylammonium, alkylacétamidoacrylate, (méth)acrylate de sulfoalkyle, acrylate de 3-sulfopropyle, méthacrylate de 3-sulfopropyle, et des sels, isomères, dérivés et mélanges de ceux-ci.

**5.** Procédé selon l'une quelconque des revendications 2 à 4, dans lequel le temps x va de 5 min à 100 min, plus préférablement de 10 min à 90 min, le plus préférablement de 20 à 60 min.

**6.** Procédé selon l'une quelconque des revendications précédentes, dans lequel, après l'application de la formulation oxydante sur les cheveux, mais avant le démouillage des cheveux, on laisse la formulation oxydante rester sur les cheveux pendant un laps de temps y, dans lequel le temps y va de 1 min à 120 min, de préférence de 2 min à 45 min, plus préférablement de 3 min à 20 min, le plus préférablement de 4 min à 10 min.

**7.** Procédé selon l'une quelconque des revendications précédentes, dans lequel la composition monomère et/ou la formulation oxydante comprennent en outre un cation et un anion, dans lequel le cation est choisi dans le groupe constitué de cations inorganiques ayant une densité de charge de 0,05 charge/picomètre ou plus.

**8.** Procédé selon l'une quelconque des revendications précédentes, dans lequel la composition monomère comprend en outre un moyen d'augmentation de viscosité.

**9.** Procédé selon la revendication 8, dans lequel le moyen d'augmentation de viscosité est un agent d'augmentation de viscosité choisi dans le groupe constitué d'épaississants non ioniques, épaississants anioniques, épaississants cationiques, épaississants amphotères et leurs mélanges.

**10.** Procédé selon l'une quelconque des revendications 8 à 9, dans lequel l'agent d'augmentation de viscosité comprend au moins un polysaccharide, de préférence au moins un hétéropolysaccharide.

**11.** Procédé selon l'une quelconque des revendications 2 à 10, dans lequel, pendant le temps x, les cheveux sont exposés à une humidité relative d'au moins 70 %, dans l'heure qui suit l'application de la composition monomère, et ladite exposition durant 10 à 90 min.

FIG. 1

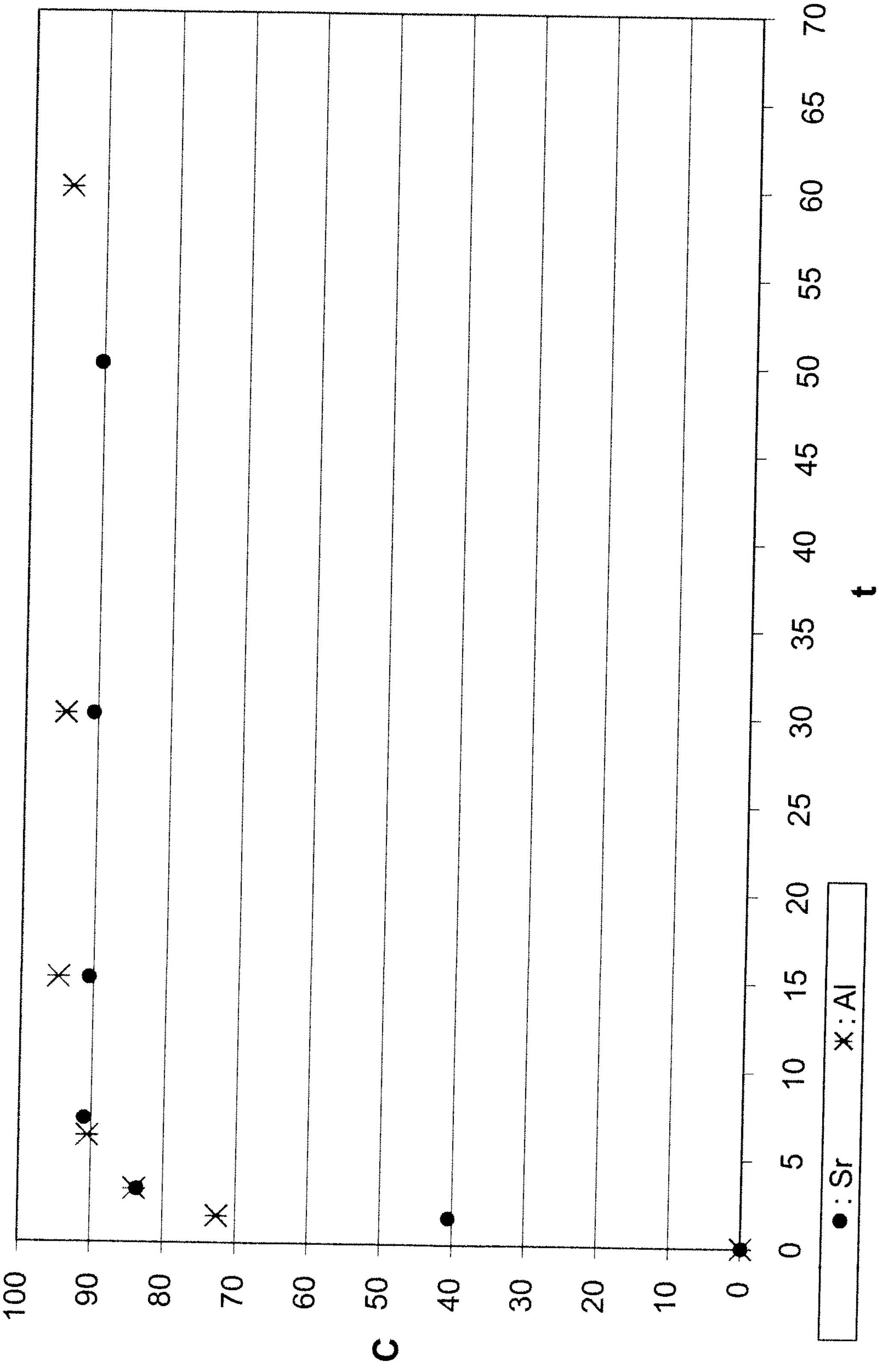
C
100
90
80
70
60
50
40
30
20
10
0
t
0
5
10
15
20
25
30
35
40
45
50
55
60
65
70
●: Sr
✳: Al

FIG. 2

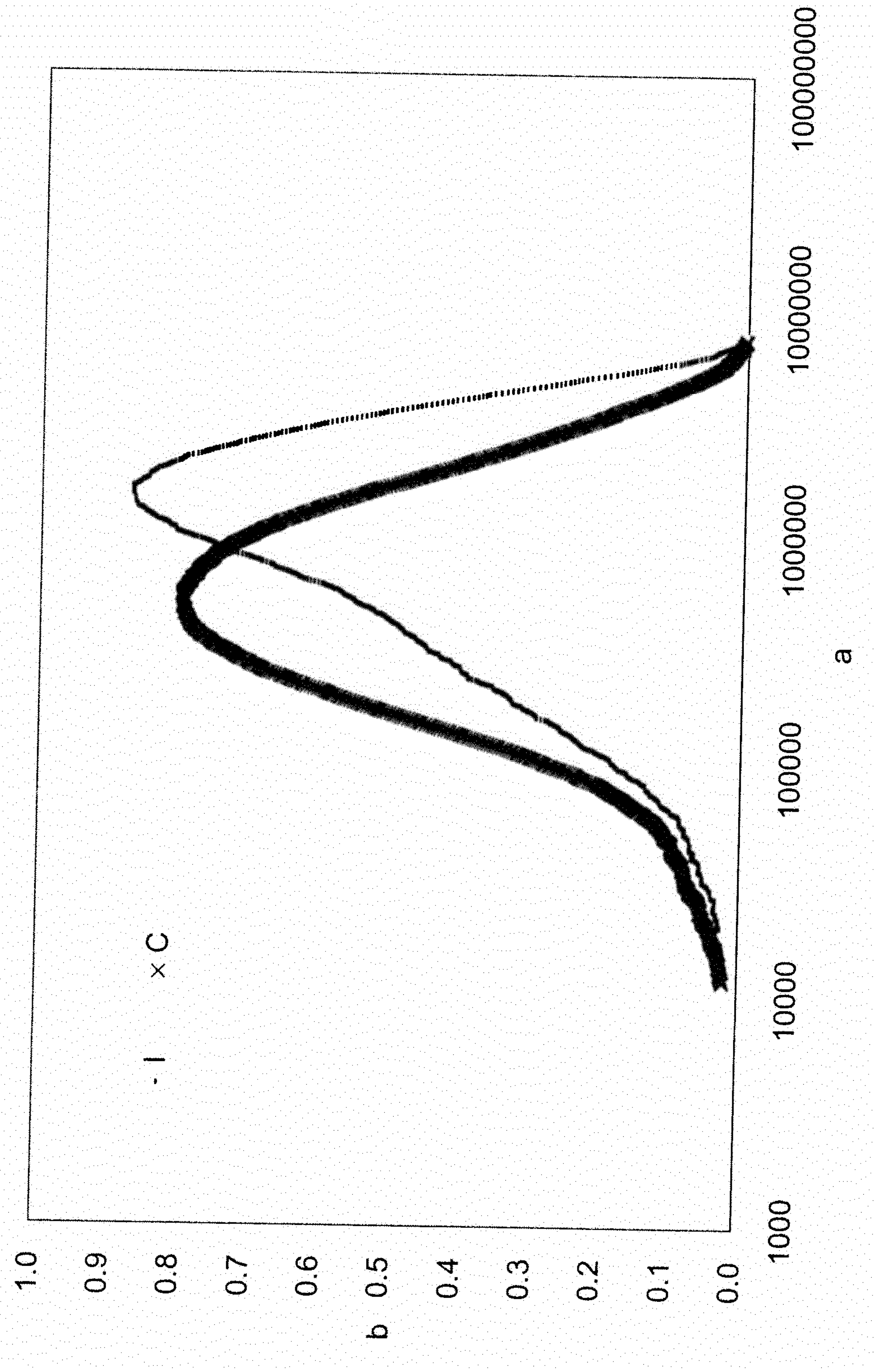
- I
× C
1.0
0.9
0.8
0.7
0.6
0.5
0.4
0.3
0.2
0.1
0.0
b
1000
10000
100000
1000000
10000000
100000000
a

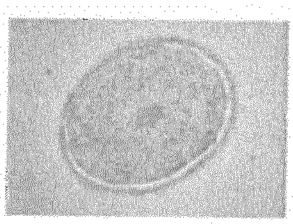

FIG. 3A

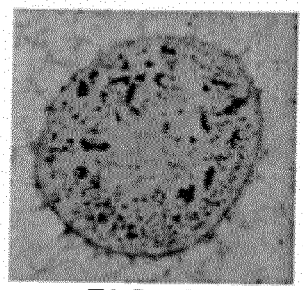

FIG. 3D

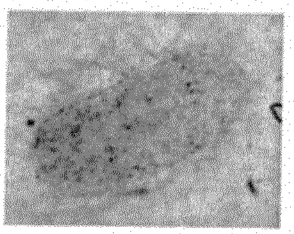

FIG. 3B

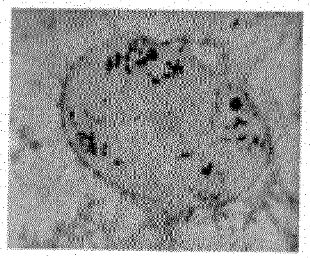

FIG. 3E

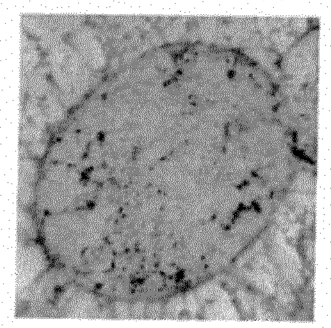

FIG. 3C

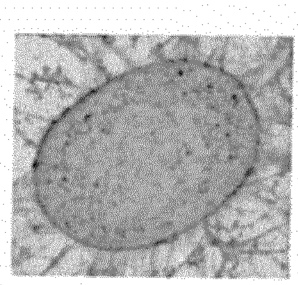

FIG. 4A

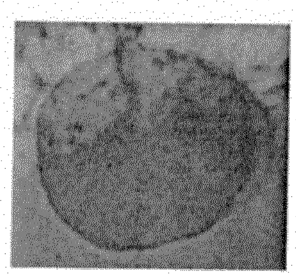

FIG. 4B

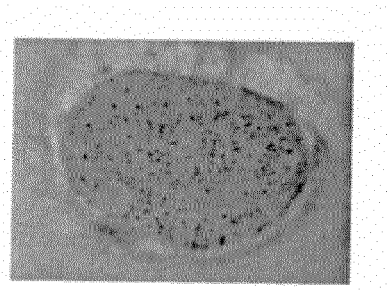

FIG. 4C

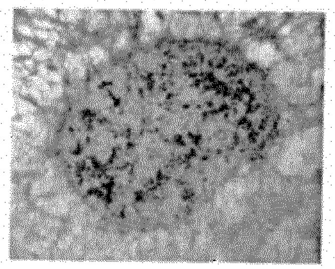

FIG. 4D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20080210253 A1
- US 7357921 B2
- US 7780742 B
- US 7740664 B
- WO 2009107062 A2
- US 5674478 A
- US 5750122 A
- US 4529586 A, Clairol
- US 4507280 A, Clairol
- US 4663158 A, Clairol
- US 4197865 L, L'Oreal
- US 4217914 A, L'Oreal
- US 4381919 A, L'Oreal
- US 4422853 A, L'Oreal
- EP 1871194 A

### Non-patent literature cited in the description

- **ATKINS P.W.** Physical Chemistry. 2001
- International Cosmetic Ingredient Dictionary. 2002
- CTFA Cosmetic Ingredient Handbook. 2004